(19) Europäisches Patentamt

European Patent Office

Office européen des brevets

(11) EP 0 372 313 B1

(12) **EUROPÄISCHE PATENTSCHRIFT**

(45) Veröffentlichungstag und Bekanntmachung des Hinweises auf die Patenterteilung:
**05.03.1997 Patentblatt 1997/10**

(21) Anmeldenummer: 89121708.5

(22) Anmeldetag: 24.11.1989

(51) Int. Cl.$^6$: **C07F 13/00**, B01J 31/16,
C07F 15/00, C07F 9/50,
C07C 5/03, C07C 45/50,
C07C 45/30, C07D 301/00,
C07F 9/53, C07C 2/86,
C07F 15/02

(54) **Sulfonierte Phenylphosphane enthaltende Komplexverbindungen**

Sulfonated phenyl phosphine-containing complexes

Complexes contenant de la phénylphosphine sulfonée

(84) Benannte Vertragsstaaten:
**AT DE ES FR GB SE**

(30) Priorität: **02.12.1988 DE 3840600**
**29.06.1989 DE 3921295**

(43) Veröffentlichungstag der Anmeldung:
**13.06.1990 Patentblatt 1990/24**

(60) Teilanmeldung: **95108099.3**
**95108100.9**

(73) Patentinhaber: HOECHST
AKTIENGESELLSCHAFT
65926 Frankfurt am Main (DE)

(72) Erfinder:
• **Herrmann, Wolfgang A., Prof.Dr.**
**D-85354 Freising (DE)**
• **Kulpe, Jürgen, Dipl.-Chem.**
**D-65929 Frankfurt (DE)**
• **Kellner, Jürgen, Dipl.-Chem.**
**D-85716 Unterschleissheim (DE)**
• **Riepl, Herbert, Cand.-Chem.**
**D-85221 Dachau (DE)**

(56) Entgegenhaltungen:
EP-A- 0 133 410      EP-A- 0 179 004
EP-A- 0 246 475      DE-B- 2 627 354
FR-A- 2 338 253      GB-A- 1 095 146
US-A- 4 522 932      US-A- 4 689 437

• **CHEMICAL ABSTRACTS, Band 110, 1989, Seite 716, Zusammenfassung Nr. 115067j, Columbus, Ohio, US; C. LARPENT et al.: "Hydrosoluble transition-metal coordination compounds of triphenylphosphine m-trisulfonate", & APPL. ORGANOMET. CHEM. 1987, 1(6), 529-34**
• **CHEMICAL ABSTRACTS, Band 105, 1986, Seite 749, Zusammenfassung Nr. 202112k, Columbus, Ohio, US; B. FONTAL et al.: "Synthesis of a new family of water- soluble clusters: Ru3(CO)12-xLx (x = 1-3), Os3(CO)10L2, and Ir4(CO)9L3 (L = trisodium salt (hydrated) of tris(m-sulfonatophenyl)phosphine)", & INORG. CHEM. 1986, 25(24), 4320-2**
• **CHEMICAL ABSTRACTS, Band 108, 1988, Seite 678, Zusammenfassung Nr. 130986k,**
• **Columbus, Ohio, US; C. LARPENT et al.: "Transition metals and a water-soluble ligand for the catalytic hydrogenation of aqueous solutions of unsaturated carboxylic acids", & C.R. ACAD. SCI., SER. 2 1987, 304(17), 1055-7**
• **CHEMICAL ABSTRACTS, Band 109, 1988, Seite 99, Zusammenfassung Nr. 8348j, Columbus, Ohio, US; H. BACH et al.: "Industrial use of water-soluble ligands for hydroformylation catalysts", & CHEM.-ING.-TECH. 1987, 59(11), 882-3**
• **CHEMICAL ABSTRACTS, Band 108, 1988, Seite 647, Zusammenfassung Nr. 186148f, Columbus, Ohio, US; P. ESCAFFRE et al.: "Water as solvent and hydrogen source for the low pressure hydroformylation reaction using rhodium complexes", & NEW J. CHEM. 1987, 11(8-9), 601-4**

- ANGEWANDTE CHEMIE INTERNATIONAL EDITION IN ENGLISH, Band 29, Nr. 4, 1990, Seiten 391-393, VCH Verlagsgesellschaft mbH, Weinheim, DE; W.A. HERRMANN et al. : "Water-soluble metal complexes of the sulfonated triphenylphosphane TPPTS:
- Preparation of the pure compounds and their use in catalysis"
- CHEMICAL ABSTRACTS, Band 107, 1987, Seite 779, Zusammenfassung Nr. 108185f, Columbus, Ohio, US; C. LARPENT et al.: "Rhodium(I) production during the oxidation by water of a hydrosoluble phosphine", & INORG. CHEM. 1987, 26(17), 2922-4
- CHEMICAL ABSTRACTS, Band 107, 1987, Seite 123, Zusammenfassung Nr. 42066j, Columbus, Ohio, US; H. BAHRMANN et al.: "Water-soluble phosphines as ligands for hydroformylation catalysts", & PHOSPHORUS SULFUR 1987, 30(3-4), 611-14
- CHEMICAL ABSTRACTS, Band 111, 1989, Seite 125, Zusammenfassung Nr. 136402a, Columbus, Ohio, US; P. KALCK: "Dinuclear rhodium(I) complexes for the selective hydroformylation of alkenes using either carbon monoxide/molecular hydrogen or carbon monoxide/water", & PURE APPL. CHEM. 1989, 61(5), 967-71
- CHEMICAL ABSTRACTS, Band 110, 1989, Seite 130, Zusammenfassung Nr. 117090k, Columbus, Ohio, US; P. KALCK et al.: "Water-soluble dinuclear rhodium complexes as catalytic precursors for the hydroformylation of alkenes in a two phases system", & NEW J. CHEM. 1988, 12(6-7), 687-90

Bemerkungen:

Die Akte enthält technische Angaben, die nach dem Eingang der Anmeldung eingereicht wurden und die nicht in dieser Patentschrift enthalten sind.

EP 0 372 313 B1

**Beschreibung**

Die Erfindung betrifft neue Komplexverbindungen von Elementen der Gruppe VIII A des Periodensystems. Gemeinsames Merkmal dieser Verbindungen ist, daß sie als Komplexligand das Trinatriumsalz von Tris(m-sulfophenyl)phosphan und gegebenenfalls weitere Liganden enthalten. Die Verbindungen sind in Wasser ohne Zersetzung löslich.

Komplexverbindungen definierter Zusammensetzung, die das Trinatriumsalz des m-trisulfonierten Triphenylphosphans der chemischen Formel $P(C_6H_4\text{-}m\text{-}SO_3Na)_3$ als einzigen oder als einen von mehreren Liganden enthalten, sind kaum bekannt. In der DE 27 00 904 C2, Beispiel 12, wird die Umsetzung von Bis(1,5-cyclooctadien)nickel mit dem Trinatriumsalz von Tris(m-sulfophenyl)phosphan (im folgenden TPPTS genannt) beschrieben. Man erhält eine rotgefärbte Verbindung, die aus ihrer wäßrigen Lösung durch Eindampfen im Vakuum als feste Substanz gewonnen wird. Nach Angaben der Autoren soll es sich bei dieser Verbindung um das Tetrakis-trinatriumsalz von Tris[(m-sulfophenyl)phosphan]nickel(O) handeln. In der gleichen Druckschrift finden sich auch pauschale Angaben über die Herstellung von TPPTS-Komplexverbindungen des Eisens und des Palladiums. Demnach soll man wasserlösliche Verbindungen oder solche Verbindungen, die unter Reaktionsbedingungen in Lösung gehen, in Gegenwart eines Reduktionsmittels mit wäßriger TPPTS-Lösung umsetzen. Als Reduktionsmittel kommen z.B. $Na[BH_4]$, $K[BH_4]$, Zinkpulver, Magnesium oder Borhydride in Betracht. Weder das Herstellungsverfahren noch einzelne Verbindungen werden durch Beispiele näher beschrieben oder gar charakterisiert.

Komplexverbindungen, die TPPTS als Liganden enthalten, ohne daß die genaue Zusammensetzung dieser Verbindungen bekannt ist, bilden sich bei verschiedenen Umsetzungen aus Metall oder Metallverbindungen, TPPTS und gegebenenfalls weiteren Liganden. So haben speziell Rhodium-Komplexe mit TPPTS-Liganden in jüngster Zeit als Bestandteil von Katalysatorsystemen Bedeutung erlangt, die bei der Hydroformylierung von Olefinen Anwendung finden. Gegenüber anderen Katalysatoren, die für dieselbe Reaktion eingesetzt werden, haben sie den Vorteil, in Wasser löslich zu sein. Daher kann die Hydroformylierung in einem aus wäßriger und organischer Phase bestehenden heterogenen Reaktionsmedium (Zweiphasensystem) durchgeführt werden mit dem Ergebnis, daß sich das Reaktionsprodukt durch einfache Phasentrennung vom wasserlöslichen Katalysator abtrennen läßt. Darüber hinaus stellt diese Arbeitsweise sicher, daß der wertvolle Edelmetallkatalysator annähernd verlustfrei wiedergewonnen oder in die Synthesestufe zurückgeführt werden kann. Ein derartiger Prozeß ist z.B. in der DE 26 27 354 B2 beschrieben.

Aus Chem. Ing.-Techn. 1987, 59, S. 882 - 3 und New. J. Chem. 1987, 11, S. 601 - 4 ist die in-situ hergestellte Komplexverbindung $RhH(CO)(P(TPPTS)_3)$ bekannt, die bei der Hydroformylierung in Kombination mit überschüssigem TPPTS als Katalysatorsystem nur unbefriedigende Ergebnisse liefert.

Auch die Anlagerung von Cyanwasserstoff an ungesättigte organische Verbindungen kann in Gegenwart einer Verbindung des nullwertigen Nickels oder des Eisens oder Palladiums in reduzierter Wertigkeitsstufe und einer wäßrigen Lösung eines sulfonierten Triphenylphosphans, insbesondere einer wäßrigen Lösung von TPPTS, als Katalysator erfolgen. Diese Arbeitsweise ist in der bereits zitierten DE 27 00 904 C2 beschrieben. Statt der Komponenten Nickelsalz und TPPTS-Lösung kann auch eine eigens hergestellte Komplexverbindung, der die Zusammensetzung $Ni(TPPTS)_4$ zugeschrieben wird, als Katalysator eingesetzt werden.

Trotz der geschilderten Vorteile der Verwendung wasserlöslicher TPPTS-Komplexverbindungen als Katalysatoren ist über ihren Einsatz bei anderen Reaktionen nichts bekannt geworden. Dieser Umstand ist wohl in erheblichem Maße darauf zurückzuführen, daß es trotz intensiver Bemühungen bisher nicht gelang, TPPTS enthaltende, wasserlösliche Komplexverbindungen in reiner Form zu isolieren und damit die Voraussetzung für ihre stoffliche Charakterisierung durch chemische und physikalische Analysenverfahren zu schaffen.

Der vorliegenden Erfindung liegt daher die Aufgabe zugrunde, TPPTS enthaltende Komplexverbindungen bestimmter Metalle in definierter, reproduzierbarer Zusammensetzung bereitzustellen.

Die Erfindung besteht in neuen Komplexverbindungen von Elementen der Gruppe VIII A des Periodensystems, ausgenommen die Verbindungen $RhCl(P(m\text{-}C_6H_4SO_3Na)_3)_3$, $RhCl(P(m\text{-}C_6H_4SO_3Na)_3)_2$, $[RhCl(P(m\text{-}C_6H_4SO_3Na)_3)_2]_2$, das Umsetzungsprodukt von Bis(1,5-cyclooctadien)nickel mit dem Trinatriumsalz von Tris(m-sulfophenyl)phosphan und Verbindungen $[Rh(\mu\text{-}S\text{-}R)(L)(P(m\text{-}C_6H_4SO_3Na)_3)]_2$, wobei die Reste R, gleich oder verschieden, ein Kohlenwasserstoff-Radikal mit gegebenenfalls einem oder mehreren Substituenten bedeuten oder auch gemeinsam durch ein einziges divalentes Radikal gebildet werden und L CO oder $P(m\text{-}C_6H_4SO_3Na)_3$ darstellt, dadurch gekennzeichnet, daß sie als Komplexligand das Trinatriumsalz von Tris(m-sulfophenyl)phosphan und gegebenenfalls weitere Liganden enthalten.

Die neuen Verbindungen können durch die allgemeine Formel

$$L^1_w L^2_x M_y(TPPTS)_z$$

wiedergegeben werden. In dieser Formel bedeuten $L^1$ und $L^2$ gleiche oder unterschiedliche Liganden, die in der Komplexverbindung neben TPPTS an das Zentralatom gebunden sein können, wie z.B. H, CO, NO, $PF_3$, S, Halogen wie Cl, ferner $\pi$-Aromatenliganden wie Cyclopentadienyl, $\pi$-Olefinliganden wie Cyclooctadien oder $\pi$-Acetylenliganden wie

Diphenylacetylen; M steht für die Elemente der Gruppe VIII A des Periodensystems als Zentralatom, insbesondere für Eisen, Ruthenium, Kobalt, Rhodium, Iridium, Nickel, Palladium oder Platin; w, x, y und z sind ganze Zahlen, wobei w und x jeweils 0 bis 7y bedeuten, y 1 bis 6 und z ≤ 4y sind.

Die neuen Verbindungen sind kristalline, zumeist farbige Substanzen. Sie sind in Wasser ohne Zersetzung löslich und können aus der wäßrigen Lösung als Hydrate in Form von Pulvern oder Kristallen isoliert werden. Diese Hydrate enthalten je Natriumion ein Molekül Wasser. Bei Raumtemperatur sind sie mehrheitlich an der Luft beständig.

Die beanspruchten Verbindungen sind präparativ auf verschiedenen Wegen zugänglich:

- durch Synthese aus einfachen Verbindungen, d.h. Salzen jenes Elements, das das Zentralatom der Komplexverbindung bildet;

- durch Ligandenaustauschreaktion aus Komplexverbindungen gemäß

$$L_w^1 L_{x+z}^2 M_y + z\ TPPTS \rightarrow L_w^1 L_x^2 M_y (TPPTS)_z + zL^2$$

wobei $L^1$, $L^2$ und M sowie x, y und z die obengenannten Bedeutungen haben, mit der Maßgabe, daß z kleiner oder gleich x ist;

- durch Einführung von TPPTS-Liganden in Komplexverbindungen, die nicht durch einfachen Ligandenaustausch im Sinne der vorstehenden Gleichung, sondern durch Eliminierungs- und/oder Substitutionsreaktionen erfolgt.

Zur Synthese aus einfachen Verbindungen geht man zweckmäßig von wasserlöslichen Salzen aus. Die Art des Anions hat im allgemeinen keinen Einfluß auf den Ablauf der Reaktion. Geeignet sind z.B. die Halogenide, insbesondere die Chloride, Salze von Sauerstoffsäuren wie Nitrate und Sulfate sowie Salze von Carbonsäuren wie Formiate und Acetate. Das in Wasser gelöste Salz wird mit einer wäßrigen TPPTS-Lösung im stöchiometrischen Verhältnis oder im Überschuß umgesetzt. Falls die Oxidationsstufe des Metalls in der Komplexverbindung niedriger als in dem Ausgangssalz ist, kann entweder überschüssiges TPPTS als Reduktionsmittel wirken oder man setzt der Reaktionslösung ein Reduktionsmittel zu. Geeignet sind z.B. Na[BH$_4$] oder Hydrazinhydrat. In diesem Fall empfiehlt es sich auch unter Luftausschluß zu arbeiten. Im allgemeinen läuft die Umsetzung bereits bei Raumtemperatur ab; nur selten muß sie durch Erhöhung der Temperatur beschleunigt oder vervollständigt werden.

Bei der Herstellung durch Ligandenaustausch setzt man als Ausgangssubstanzen Komplexverbindungen der jeweiligen Metalle ein. Entsprechend ihrer Löslichkeit werden sie in Wasser oder in einem organischen Lösungsmittel wie aromatischen Kohlenwasserstoffen (z.B. Toluol), Halogenkohlenwasserstoffen (z.B. Chloroform), Alkoholen (z.B. Ethanol) oder heterocyclischen Verbindungen (z.B. Tetrahydrofuran) gelöst. TPPTS wird wiederum in Form einer wäßrigen Lösung verwendet und im stöchiometrischen Verhältnis oder im Überschuß eingesetzt.

Auch im Falle der Synthese durch Ligandenaustausch genügt es, bei Raumtemperatur zu arbeiten. Selbst wenn die Ausgangskomplexverbindung in einem mit Wasser nicht mischbaren organischen Lösungsmittel gelöst ist, die Umsetzung also in einem flüssigen Zweiphasensystem abläuft, reichen kurze Reaktionszeiten aus; intensive Rührung fördert hierbei die Reaktion.

Die Herstellung der neuen Komplexverbindungen durch Eliminierungs- und Substitutionsreaktionen erfolgt in ähnlicher Weise und unter vergleichbaren Bedingungen wie bei der Synthese durch Ligandenaustausch. Weitere Liganden führt man in die Komplexverbindungen in bekannter Weise ein, z.B. durch Einleiten von CO oder durch Zugabe einer den Nitrosylrest, eine Schwefel- oder Hydridgruppe abspaltenden Verbindung.

Zur Aufarbeitung des Reaktionsproduktes und zur Isolierung der neuen Verbindungen, die unabhängig vom Herstellungsverfahren, in wäßriger Lösung vorliegen, dampft man das Wasser, gegebenenfalls nach vorheriger Filtration der Lösung, im Vakuum ab. Im allgemeinen erhält man auf diesem Wege nicht die reinen Verbindungen, sondern verunreinigte Produkte oder auch Gemische verschiedener TPPTS-Komplexverbindungen, die sich bei der Herstellung nebeneinander gebildet haben. Es ist daher erforderlich, zur Gewinnung der Reinsubstanzen spezielle Reinigungs- und Trennverfahren anzuwenden. Als besonders geeignet zur Lösung dieser Aufgabe hat sich die Chromatographie an Gelen erwiesen, die Gegenstand der deutschen Patentanmeldung P 38 22 036.9 ist. Angepaßt an die Eigenschaften der jeweiligen Verbindung, muß hierbei gegebenenfalls auf Licht- oder Luftausschluß geachtet werden; auch die Elutionsmittel und -geschwindigkeiten richten sich nach dem jeweiligen Reinigungs- bzw. Trennproblem. Nach dieser Behandlung liegen die Verbindungen analysenrein und spektroskopisch rein vor.

Wie bereits weiter oben gesagt wurde, kristallisieren die neuen Verbindungen aus wäßriger Lösung als Hydrate. Durch Entwässerung unter schonenden Bedingungen, d.h. bei Temperaturen unterhalb des Schmelz- bzw. Zersetzungspunktes und unter Anwendung von vermindertem Druck, zweckmäßig Hochvakuum, lassen sich aus ihnen, ohne daß Zersetzung eintritt, die wasserfreien Verbindungen herstellen. Der beanspruchte Schutz erstreckt sich daher sowohl auf die wasserhaltigen als auch auf die wasserfreien TPPTS-Komplexe.

Die erfindungsgemäßen Verbindungen sind katalytisch aktiv und werden mit Erfolg als Katalysatoren oder Bestandteile von Katalysatoren bei verschiedenen Reaktionen eingesetzt.

Besonders hervorzuheben ist, daß durch Einsatz der reinen Verbindungen ausbeutemindernde Neben- und Folgereaktionen vermieden werden, die häufig dann auftreten, wenn der Katalysator im Reaktionsgemisch "in situ" gebildet wird. Dieser Sachverhalt beruht darauf, daß die "in situ-Herstellung" im allgemeinen mit der Bildung unwirksamer oder störender Nebenprodukte verbunden ist. In den von anderen Autoren beschriebenen Fällen enthalten solche Katalysatoren ausnahmslos noch freies, überschüssiges TPPTS, welches die Reaktivität der eigentlichen TPPTS-Komplexverbindung stark verändert. Die Verwendung reiner TPPTS-Komplexverbindungen hat gezeigt, daß diese Verbindungsklasse in viel umfangreicherem Maße katalytisch wirksam ist, als bisher bekannt und zu erwarten war.

So sind die erfindungsgemäßen Komplexverbindungen ausgezeichnete Hydrierkatalysatoren. Beispielsweise werden sie mit Erfolg für die Hydrierung von Olefinen zu gesättigten Kohlenwasserstoffen eingesetzt. Die Reaktion verläuft in ihrer Gegenwart schon bei Normaldruck und Temperaturen zwischen 20 und 40 °C.

Auch die Verschiebung des Wassergasgleichgewichts

$$CO + H_2O \rightleftharpoons CO_2 + H_2$$

in Richtung auf Wasserstoff- und Kohlendioxidbildung wird durch die neuen Verbindungen bewirkt. Sie ermöglichen es, die Umsetzung bereits bei Raumtemperatur und etwa 1,5 MPa Druck durchzuführen. Auf diesem Wege gebildeter Wasserstoff kann für katalytische Hydrierungen, z.B. die Umsetzung organischer Nitroverbindungen zu organischen Aminen, genutzt werden. Da bei der obigen Reaktion neben Wasserstoff immer Kohlenmonoxid vorhanden ist, kann das Gasgemisch auch zur Hydroformylierung von Olefinen verwendet werden.

Die Wasserstofferzeugung aus Wasser und Kohlenmonoxid in Gegenwart der erfindungsgemäßen Verbindungen als Katalysatoren ermöglicht auch die Hydrocarbonylierung von Olefinen. Besonders bemerkenswert ist, daß die Reaktion schon bei relativ niedrigen Temperaturen abläuft. So erhält man z.B. aus Ethylen bei 140 bis 150 °C nach der Gleichung

$$H_2C{=}CH_2 + CO + H_2 \rightarrow H_5C_2 - CO - C_2H_5$$

Diethylketon.

Weiterhin können die neuen Komplexverbindungen als Katalysatoren für die Hydroformylierung olefinisch ungesättigter Verbindung verwendet werden. Sie haben sich sowohl bei der Umsetzung linearer und cyclischer Olefine als auch bei der Umsetzung von Verbindungen bewährt, die neben einer Doppelbindung auch funktionelle Gruppen im Molekül enthalten.

Auch die Oxidation unterschiedlicher Verbindungsklassen wird durch die neuen, TPPTS als Komplexligand enthaltenden Verbindungen katalysiert. So erhält man mit Iodosobenzol als Oxidationsmittel aus sekundären Alkoholen die entsprechenden Ketone, aus Olefinen Epoxide und aus Alkinen Diketone.

Die erfindungsgemäßen Komplexverbindungen katalysieren darüber hinaus Reaktionen, bei denen Kohlenstoff-Kohlenstoff-Bindungen neu gebildet werden. Ein Beispiel für diesen Reaktionstyp ist die Allen-Alkin-Kupplung gemäß

$$R^1R^2C{=}C{=}CHX + HC{\equiv}CR^3 \rightarrow R^1R^2C{=}C{=}CH{-}C{\equiv}CR^3 + HX$$

wobei $R^1$, $R^2$, $R^3$ Alkyl- und/oder Arylgruppen, $R^1$ auch H, sind. Exemplarisch für diese Umsetzung ist die Reaktion von 1-Bromallen mit Phenylacetylen, die unter der katalytischen Einwirkung der neuen Verbindungen schon bei Raumtemperatur vor sich geht.

Schließlich katalysieren die erfindungsgemäßen Komplexverbindungen die Addition von sekundären organischen Aminen an Kohlenstoff-Kohlenstoff-Doppelbindungen nach dem Schema

$$R^1R^2C{=}CH_2 + HNR^3 \rightarrow R^1R^2CH{-}CH_2{-}NR^3$$

Die Substituenten $R^1$, $R^2$ und $R^3$ haben die oben wiedergegebene Bedeutung.

In den folgenden Beispielen sind Herstellung und Eigenschaften der neuen Verbindungen beschrieben.

TPPTS wurde nach dem in der DE 32 35 030 A1 beschriebenen Verfahren hergestellt und gereinigt. $OP(C_6H_4\text{-}m\text{-}SO_3Na)_3$, im folgenden TPPOTS genannt, wird durch Gelchromatographie nach dem in der deutschen Patentanmeldung P 38 22 036.9 beschriebenen Verfahren abgetrennt. Die angegebenen Ausbeuten beziehen sich auf die gereinigten Substanzen.

Bei den zur Charakterisierung der neuen Substanzen herangezogenen NMR- und IR-Daten wurden folgende Abkürzungen verwendet:
s = Singulett,    d = Dublett,    t = Triplett,
m = Multiplett
ss = sehr schwach, s = schwach, m = mittelstark,

st = stark, sst = sehr stark, b = breite Bandenform,
Sch = Schulter.

Die zur chromatographischen Reinigung der neuen Substanzen verwendeten Sephadex-Gele sind mit Epichlorhydrin vernetzte Dextrane. Bei Fractogel handelt es sich um Oligoethylglykol-Glycydyl/Methacrylat/Pentaerythroldimethacrylat-Copolymerisat.

### Beispiel 2: Synthese von $Fe(CO)_3(TPPTS)_2 \cdot 6 H_2O$ und $Fe(CO)_4(TPPTS) \cdot 3 H_2O$

470 mg (1.3 mmol) Enneacarbonyldieisen, $Fe_2(CO)_9$, werden mit einer Lösung von 587 mg (1.3 mmol) TPPTS in 50 ml destilliertem Wasser 30 min unter Rückfluß gekocht. Es entsteht eine orangefarbene Lösung, die vom ebenfalls entstandenen, grünen Dodecacarbonyltrieisen, $Fe_3(CO)_{12}$, abfiltriert wird. Das Filtrat wird auf 10 ml eingeengt und über eine Sephadex-G-15-Säule chromatographiert. Es entwickeln sich fünf Zonen, von denen die ersten beiden aufgefangen werden:

1. Fraktion: $Fe(CO)_3(TPPTS)_2 \cdot 6 H_2O$, gelber Feststoff. Ausbeute 669 mg (38 %).

### Charakterisierung

$^{31}$P-NMR (109.3 MHz, $D_2O$, +28 °C): $\delta$ = 74.76 ppm
IR (cm$^{-1}$, KBr): 1885 sst (vCO)

| Elementaranalyse ($C_{39}H_{36}FeNa_6O_2P_2S_6$; 1383.28) | | | |
|---|---|---|---|
| Ber. | C 31.36 | H 2.86 | Fe 4.00 | P 4.50 |
| Gef. | C 33.80 | H 2.80 | Fe 3.42 | P 4.09 |

2. Fraktion: $Fe(CO)_4(TPPTS) \cdot 3 H_3O$, orangefarbener Feststoff. Ausbeute: 167 mg (16 %).

### Charakterisierung

$^{31}$P-NMR (109.3 MHz, $D_2O$, +28 °C): $\delta$ = 84.95 ppm
IR (cm$^{-1}$, KBr): 2050 sst, 1977 sst, 1944 sst (vCO)

| Elementaranalyse ($C_{22}H_{18}FeNa_3O_{16}PS_3$; 789.49) | | | |
|---|---|---|---|
| Ber. | C 35.88 | H 2.27 | P 3.92 | Fe 7.06 |
| Gef. | C 34.30 | H 2.46 | P 4.40 | Fe 6.90 |

### Beispiel 3: Synthese von $Ru(NO)_2(TPPTS)_2 \cdot 6 H_2O$

Variante A. Eine siedende Lösung von 1.71 g (3 mmol) TPPTS in 25 ml Ethanol und 15 ml Wasser wird mit 0.13 g (0.5 mmol) $RuCl_3 \cdot 3 H_2O$ in 10 ml Ethanol versetzt. Dazu tropft man langsam eine Lösung von 100 mg (2.6 mmol) $Na[BH_4]$ in 10 ml Ethanol, bis die Lösung tiefviolett gefärbt ist (etwa 1/3 der Lösung). Anschließend werden 210 mg (1.0 mmol) Diazald$^{®}$ (N-Methyl-N-nitroso-p-toluolsulfonamid), gelöst in 10 ml Ethanol, und die restliche Natriumboranat-Lösung schnell zugegeben. Man läßt noch 10 min unter Rückfluß kochen und kühlt dann auf Raumtemperatur ab. Es fällt ein rotbrauner Niederschlag aus, der über eine Glasfritte abfiltriert, mit Ethanol gewaschen und durch Säulenchromatographie an Sephadex G-15 gereinigt wird. Aus einer ersten, grauschwarzen Zone isoliert man eine schwarze Substanz (5 mg), deren IR-Spektrum neben ν(SO)-Schwingungen von TPPTS noch Banden bei 1961 (s) und 1847 (m) enthält. Aus einer dicht darauf folgenden roten Zone gewinnt man den gewünschten Ruthenium-Komplex. Ausbeute: 370 mg (53 %); rote Kristalle.

Variante B. Zu einer siedenden Lösung von 1.71 g (3 mmol) TPPTS in 20 ml Ethanol und 10 ml Wasser gibt man 0.13 g (0.5 mmol) RuCl$_3$ · 3 H$_2$O in 10 ml Ethanol, 2 ml Triethylamin und 200 mg Diazald$^R$ in 10 ml Ethanol. Nach Zusatz weiterer 3 ml Triethylamin wird das Reaktionsgemisch noch 5 min unter Rückfluß gekocht. Darauf läßt man auf Raumtemperatur abkühlen, filtriert über eine Glasfritte und zieht vom Filtrat das Lösungsmittel im Vakuum ab. Das Rohprodukt wird durch Säulenchromatographie an Sephadex G-15 gereinigt. Ausbeute: 430 mg (62 %); rote Kristalle.

Charakterisierung

$^{31}$P-NMR (109.3 MHz, D$_2$O, 5 °C): δ = 56.0 ppm (s).
IR (KBr, cm$^{-1}$): ν(SO) = 1224 (Sch, sst), 1199 (sst), 1040 (sst), 624 (sst); ν(NO) = 1675 (st).

| Elementaranalyse (C$_{36}$H$_{36}$N$_2$Na$_6$O$_{26}$P$_2$RuS$_6$; 1405.99) | | | | | | | |
|------|---------|--------|--------|---------|--------|---------|----------|
| Ber. | C 30.75 | H 2.58 | N 1.99 | O 29.59 | P 4.41 | Ru 7.19 | S 13.68 |
| Gef. | C 30.56 | H 2.76 | N 1.68 | O 28.84 | P 4.11 | Ru 7.01 | S 14.72 |

Beispiel 4: Synthese von RuCl$_2$(TPPTS)$_2$ · 6 H$_2$O

Variante A: Bei Raumtemperatur gibt man zu 130 mg (0.5 mmol) Ruthenium(III)-chlorid-Trihydrat, RuCl$_3$ · 3 H$_2$O, unter kräftigem Rühren 1.42 g (2.5 mmol) TPPTS in 15 ml Wasser, erhitzt sodann auf 50 °C (Badtemperatur) und läßt bei dieser Temperatur 24 h reagieren. Anschließend wird von der klaren, braunen Lösung das Wasser im Vakuum einer Ölpumpe abgezogen. Der Rückstand wird durch Säulenchromatographie an Sephadex G-15 gereinigt. Ausbeute: 430 mg (61 %); braune Kristalle.

Variante B: Zu einer Lösung von 120 mg (0.2 mmol) Dichlorotetrakis(triphenylphosphan)ruthenium RuCl$_2$[P(C$_6$H$_5$)$_3$]$_4$ in 20 ml Toluol gibt man 570 mg (1 mmol) TPPTS in 10 ml Wasser und läßt das entstehende Zweiphasensystem 15 h bei Raumtemperatur rühren. Nach Trennung der Phasen wird die organische Phase zweimal mit je 5 ml Wasser gewaschen. Die vereinigten wäßrigen Phasen werden zweimal mit je 5 ml Toluol extrahiert. Anschließend zieht man das Wasser im Vakuum einer Ölpumpe ab. Das Rohprodukt wird durch Säulenchromatographie an Sephadex G-15 gereinigt. Ausbeute: 160 mg (57 %); braune Kristalle.

Charakterisierung

$^{31}$P-NMR (109.3 MHz, D$_2$O, 5 °C): δ = 57.0 ppm (s).
IR (KBr, cm$^{-1}$): ν(SO) = 1223 (Sch, sst), 1198 (sst), 1039 (sst)

| Elementaranalyse (C$_{36}$H$_{36}$Cl$_2$Na$_6$O$_{24}$P$_2$RuS$_6$; 1416.89) | | | |
|------|---------|--------|---------|
| Ber. | C 30.52 | H 2.56 | P 4.37 | S 13.58 |
| Gef. | C 31.12 | H 2.84 | P 4.21 | S 14.04 |

Beispiel 5: Synthese von $Co_2(CO)_6(TPPTS)_2 \cdot 6 H_2O$

100 mg (0.3 mmol) Octacarbonyldicobalt, $Co_2(CO)_8$, werden in 10 ml Toluol gelöst. Zu der Lösung gibt man 400 mg (0.7 mmol) TPPTS in 10 ml Wasser und läßt 3 h bei Raumtemperatur rühren. Nach Trennung der Phasen wäscht man die organische Phase zweimal mit je 5 ml Wasser und die vereinigten wäßrigen Phasen zweimal mit je 5 ml Toluol. Das Wasser wird im Vakuum abgezogen und das Rohprodukt durch Säulenchromatographie an Sephadex G-25 gereinigt. Ausbeute: 370 mg (81 %); braunes Pulver.

Charakterisierung

$^{31}P$-NMR    (109.3 MHz, $D_2O$, 5 °C): $\delta$ = 68.8 ppm (s).

IR    ($cm^{-1}$, KBr): v(CO) = 1954 (sst); v(SO) = 1224 (Sch, sst), 1200 (sst), 1039 (sst), 623 (sst).

| Elementaranalyse ($C_{42}H_{36}Co_2Na_6P_2O_{30}S_6$; 1530.84) | | | | |
|---|---|---|---|---|
| Ber. | C 32.95 | H 2.37 | O 31.35 | P 4.05 | S 12.57 |
| Gef. | C 32.44 | H 2.37 | O 31.25 | P 3.97 | S 12.13 |

Beispiel 6: Synthese von $CoH(CO)(TPPTS)_3 \cdot 9 H_2O$

Eine Lösung von 120 mg (0.5 mmol) $CoCl_2 \cdot 5 H_2O$ in 5 ml destilliertem Wasser wird mit 1.64 g (3 mmol) TPPTS versetzt und dann auf 5 °C gekühlt. Nachdem sich die Feststoffe unter Rühren gelöst haben, tropft man innerhalb 1 h eine Lösung von 32 mg (0.9 mmol) $Na[BH_4]$ in 20 ml destilliertem Wasser zu und leitet gleichzeitig Kohlenmonoxid ein. Die gelbe Lösung wird im Vakuum auf ein Viertel des ursprünglichen Volumens eingeengt und an einer Sephadex G-15-Säule chromatographiert. Die Substanz ist kaum luftempfindlich. Ausbeute: 856 mg (89 %); kanariengelbes Pulver.

Charakterisierung

$^{31}P$-NMR    (109.3 MHz, $D_2O$, 5 °C): $\delta$ = 45.93 ppm

$^1H$-NMR    (270 $NH_3$, $D_2O$, 5 °C): $\delta$ = 7,32 ppm [m, 27H], $\delta$ = 7.29 [br, 9H], $\delta$ = -12.35 [q, $^2J(P,H)$ = 45Hz, 1H]

IR    ($cm^{-1}$, KBr): 1953 sst (vCoH), 1904 sst (vCO)

| Elementaranalyse ($C_{55}H_{55}CoNa_9O_{37}P_3S_9$; 1924.99) | | | |
|---|---|---|---|
| Ber. | C 34.20 | H 2.80 | Co 3.06 | P 4.80 |
| Gef. | C 34.07 | H 2.87 | Co 3.09 | P 4.76 |

Beispiel 7: Synthese von $CoH_2(TPPTS)_2 \cdot 9 H_2O$

Eine Lösung von 120 mg (0.5 mmol) $CoCl_2 \cdot 5 H_2O$ in 5 ml destilliertem Wasser wird bei 5 °C mit 1.64 (3 mmol) TPPTS versetzt. Anschließend tropft man unter Rühren innerhalb 1 h eine Lösung von 32 mg (0.9 mmol) $Na[BH_4]$ in 40 ml destilliertem Wasser zu. Dann engt man die Lösung bei 5 °C im Vakuum auf etwa 10 ml ein und chromatographiert über eine 15 cm lange Sephadex-G-15-Säule. Man arbeitet mit hoher Flußrate (etwa 3 - 4 Tropfen/sec) und fängt den ersten Anteil der roten Fraktion solange auf, bis die Zonenfarbe ins Grüne übergeht. Die Substanz ist im trockenen Zustand bei Normaltemperatur, in wäßriger Lösung jedoch nur um 5 °C längere Zeit haltbar. Ausbeute: 0.47 g (50 %); glasartiges, rotes Pulver.

Charakterisierung

$^{31}$P-NMR (109.3 MHz, $D_2O$, +28 °C): $\delta$ = 48.3 ppm (breit)
$^1$H-NMR (270 MHz, $D_2O$): (CoH) = -12 ppm (sehr breit)
IR (cm$^{-1}$, KBr): 2008 sst (vCoH)

Beispiel 8: Synthese von $Co_2$ $(CO)_4(H_5C_6-C\equiv C-C_6H_5)(TPPTS)_2$ · 6 $H_2O$

Zu 250 mg (0.5 mmol) ($\mu$, $\eta^2$-Diphenylacetylen)hexacarbonyl-dicobalt in 20 ml Ethanol gibt man 570 mg (1 mmol) TPPTS in 10 ml Wasser und läßt 15 h unter Rückfluß sieden. Nach Abkühlen auf Raumtemperatur zieht man das Lösungsmittel im Vakuum einer Ölpumpe ab, nimmt in 10 ml Wasser auf, filtriert über eine Schlenkfritte und zieht das Wasser im Vakuum einer Ölpumpe ab. Der schwarzbraune Rückstand wird durch Säulenchromatographie an Sephadex G-15 gereinigt. Ausbeute: 610 mg (74 %); schwarzbraunes Pulver.

Charakterisierung

$^{31}$P-NMR (109.3 MHz, $D_2O$, 5 °C): $\delta$ = 51.8 ppm (s)
IR (KBr, cm$^{-1}$): v(CO) = 2017 (sst), 1960 (sst)
v(SO) = 1220 (Sch, sst), 1195 (sst), 1039 (sst)

| Elementaranalyse ($C_{54}H_{46}Co_2Na_6O_{28}P_2S_6$; 1653.05) | | | | |
|---|---|---|---|---|
| Ber. | C 39.24 | H 2.80 | O 27.10 | P 3.75 |
| Gef. | C 39.55 | H 2.67 | O 27.16 | P 3.68 |

Beispiel 9: Synthese von RhCl(TPPTS)$_3$ · 9 $H_2O$

Variante A. Eine Lösung von 260 mg (1.0 mmol) RhCl$_3$ · 3 $H_2O$ in 20 ml Wasser wird nach Zugabe von 5.68 g (10 mmol) TPPTS gelöst in 10 ml Wasser etwa 15 h gerührt. Die entstandene Lösung, die noch freies Phosphan (TPPTS), Tris(m-sulfophenyl)phosphanoxid (TPPOTS) sowie geringe Mengen des zweikernigen Komplexes [($\mu$-Cl)Rh(TPPTS)$_2$]$_2$ enthält, wird durch Säulenchromatographie an Sephadex G-15 gereinigt. Ausbeute: 1.46 g (73 %).

Variante B. In einem Schlenkrohr werden 100 mg (0.11 mmol) Chlorotris(triphenylphosphan)rhodium(I), RhCl[P($C_6H_5$)$_3$]$_3$, in einem Gemisch aus 20 ml Toluol und 10 ml Tetrahydrofuran gelöst. Diese Lösung wird mit 20 ml einer wäßrigen Lösung von 1.87 g (3.3 mmol) TPPTS unterschichtet. Nach 12 h kräftigem Rühren wäscht man die wäßrige Phase des Zweiphasensystems zweimal mit je 5 ml Methylenchlorid. Die reine Verbindung erhält man durch Säulenchromatographie an Sephadex G-15. Ausbeute: 180 mg (82 %).

Variante C. 100 mg (0.4 mmol) Bis[($\mu$-chloro){1,2:5,6-$\eta^4$-cyclooctadien(1.5)}rhodium], [RhCl($\eta^4$-$C_8H_{12}$)]$_2$), werden in 10 ml Methylenchlorid gelöst. Hierzu gibt man 0.68 g (1.2 mmol) TPPTS in 10 ml Wasser. Das Zweiphasensystem wird 30 min kräftig gerührt. Danach isoliert man die wäßrige Phase und extrahiert die organische Phase zweimal mit je 5 ml Wasser. Die vereinigten wäßrigen Phasen wäscht man dann zweimal mit je 5 ml Methylenchlorid. Das nach Abziehen des Lösungsmittels erhaltene Rohprodukt ist für die meisten Umsetzungen genügend rein. Es kann je nach Stöchiometrie geringe Mengen TPPTS bzw. ($\eta^4$-$C_8H_{12}$)Rh$_2$($\mu$-Cl)$_2$(TPPTS)$_2$ enthalten, die sich durch Säulenchromatographie an Sephadex G-15 entfernen lassen. Ausbeute: 740 mg (93 %); roter glasartiger Feststoff.

Charakterisierung

$^{31}$P-NMR (109.3 MHz, $D_2O$, 5 °C) : $\delta$ = 34.6 ppm (dd) $^1$J(Rh,P$_A$) = 144.1 Hz
$\delta$ = 52.8 ppm (dt) [$^1$J(Rh,P$_B$) = 195.8 Hz; $^2$J(P$_A$,P$_B$) = 40.6 Hz]
IR (cm$^{-1}$, KBr) v(SO) 1206 (Sch, sst), 1181 (sst), 1027 (sst), 741 (sst), 545 (sst), 490 (sst)

| Elementaranalyse $(C_{54}H_{54}ClNa_9O_{36}P_3Rh\,S_9;\,2005.71)$ | | | |
|---|---|---|---|
| Ber. | C 32.34 | H 2.70 | P 4.63 |
| Gef. | C 32.30 | H 2.71 | P 4.60 |

### Beispiel 10: Synthese von Rh(NO)(TPPTS)$_3$ · 9 H$_2$O

Variante A. 0.37 g (0.4 mmol) Rh(NO)[P(C$_6$H$_5$)$_3$]$_3$ werden in 40 ml Toluol gelöst. Dazu gibt man 2.27 g (4 mmol) TPPTS in 20 ml Wasser und läßt das resultierende Zweiphasensystem bei 25 °C 24 h rühren. Nach dieser Zeit hat sich die organische Phase weitgehend entfärbt. Man trennt die Phasen und wäscht die organische Phase zweimal mit je 5 ml Wasser. Die vereinigten wäßrigen Phasen werden filtriert. Anschließend dampft man das Wasser im Vakuum ab. Der Rückstand wird durch Säulenchromatographie an Sephadex G-25 gereinigt. Ausbeute: 0.58 g (73 %); dunkelrote Kristalle.

Variante B. Zu einer siedenden Lösung von 5.68 g (10 mmol) TPPTS in einem Gemisch aus 40 ml Wasser und 40 ml Ethanol gibt man schnell hintereinander 0.26 g (1 mmol) RhCl$_3$.3 H$_2$O in 20 ml Ethanol, 0.40 g (1.9 mmol) Diazald$^R$ in 20 ml Ethanol und 0.30 g (7 mmol) Natriumhydroxid in 10 ml Wasser und 10 ml Ethanol. Darauf läßt man 15 min unter Rückfluß kochen und kühlt auf Raumtemperatur ab. Nach vorsichtiger Neutralisierung der alkalischen Lösung mit konzentrierter Schwefelsäure wird das Wasser im Vakuum abgedampft. Der Rückstand wird zweimal mit je 10 ml Ethanol gewaschen. Die Reinigung des Rohproduktes erfolgt durch Säulenchromatographie an Sephadex G-25. Ausbeute: 1.51 g (76 %); dunkelrote Kristalle.

Variante C. Zu einer Suspension von 220 mg (0.3 mmol) Rh(NO)Cl$_2$[P(C$_6$H$_5$)$_3$]$_2$ in 15 ml Methylenchlorid gibt man 1.14 g (2 mmol) TPPTS in 10 ml Wasser und läßt das resultierende Dreiphasensystem 20 h bei 25 °C rühren. Danach ist der Rhodiumkomplex vollständig in Lösung gegangen, und die organische Phase hat sich weitgehend entfärbt. Nach Trennung der Phasen wäscht man die organische Phase zweimal mit je 5 ml Wasser und die vereinigten wäßrigen Phasen zweimal mit je 5 ml Methylenchlorid. Anschließend dampft man das Wasser im Vakuum ab. Das Rohprodukt wird durch Säulenchromatographie an Sephadex G-25 gereinigt. Ausbeute: 420 mg (70 %); dunkelrote Kristalle.

Charakterisierung

$^{31}$P-NMR  (109.3 MHz, D$_2$O, 5 °C); δ = 48.4 ppm (d) [$^1$J(Rh,P) = 176.6 Hz].
IR        (cm$^{-1}$; KBr): ν(SO) = 1225 (Sch, sst), 1200 (sst), 1039 (sst), 623 (sst).

| Elementaranalyse $(C_{54}H_{54}Na_9NO_{37}P_3RhS_9;\,2000.28)$ | | | | | | |
|---|---|---|---|---|---|---|
| Ber. | C 32.43 | H 2.72 | N 0.70 | O 29.60 | P 4.65 | Rh 5.14 | S 14.42 |
| Gef. | C 32.61 | H 2.68 | N 0.60 | O 30.90 | P 4.54 | Rh 5.0 | S 14.23 |

### Beispiel 11: Synthese von Rh(CH$_3$COO)(TPPTS)$_3$ · 9 H$_2$O

Zu einer Lösung von 100 mg (0.11 mmol) Rh(CH$_3$COO)[P(C$_6$H$_5$)$_3$]$_3$ in 10 ml Methylenchlorid gibt man eine Lösung von 600 mg (1.05 mmol) TPPTS in 10 ml Wasser. Nach 24 h Rühren isoliert man die wäßrige Phase und chromatographiert deren Inhalt an einer mit Sephadex G-15 beschickten Säule. Ausbeute: 30 mg (15 %); rotes Pulver.

Charakterisierung

$^{31}$P-NMR (109.3 MHz, D$_2$O, 5 °C) : δ = 34.5 ppm (dd) [$^1$J(Rh,P$_A$) = 144.1 Hz]
δ = 53.0 ppm (dt) [$^1$J(Rh,P$_B$) = 195.3 Hz; $^2$J(P$_A$,P$_B$) = 40.9 Hz]

| Elementaranalyse (C$_{56}$H$_{57}$Na$_9$O$_{38}$P$_3$Rh S$_9$: 2029.30) | | | | |
|---|---|---|---|---|
| Ber. | C 33.15 | H 2.83 | Na 10.20 | Rh 5.07 |
| Gef. | C 33.15 | H 2.80 | Na 10.00 | Rh 5.10 |

### Beispiel 12: Synthese von Rh(CO)(OH)(TPPTS)$_2$ · 6 H$_2$O

Variante A: Zu einer Lösung von 2.2 g (3.88 mmol) TPPTS werden unter starkem Rühren 100 mg (0.39 mmol) festes [Rh(CO)$_2$acac] gegeben (acac = Acetylacetonat). Nach 12 h wird im Vakuum zu Trockene eingedampft. Der Rückstand wird in wenig Wasser aufgenommen und an Sephadex G-15 chromatographisch gereinigt. Ausbeute: 310 mg (58 %); rotbraunes Glas.
Variante B: Eine Lösung von 250 mg (0.12 mmol) RhH(CO)(TPPTS)$_3$ · 9 H$_2$O in 20 ml Wasser wird 12 h unter Rückfluß zum Sieden erhitzt. Anschließend wird das so erhaltene Reaktionsgemisch durch Säulenchromatographie an Sephadex G-15 getrennt. Ausbeute: 110 mg (63 %); rotbraunes Glas.

Charakterisierung:

$^{31}$P-NMR (109.3 MHz, D$_2$O, 21 °C) : δ = 31,8 ppm (d) [$^1$J(Rh,P) = 128.5 Hz]
IR (KBr, cm$^{-1}$) : v(CO) 1989, v(SO) 1196 (Sch, st) 1040 (sst) 994 (st), 791 (st), 690 (m)

| Elementaranalyse (C$_{37}$H$_{37}$Na$_6$O$_{26}$P$_2$RhS$_6$; 1392.82) | | | | | | |
|---|---|---|---|---|---|---|
| Ber. | C 31.90 | H 2.68 | O 29.86 | S 13.81 | P 4.45 | Rh 7.38 |
| Gef. | C 32.56 | H 2.73 | O 30.13 | S 14.57 | P 4.38 | Rh 7.21 |

### Beispiel 13: Synthese von Rh(CO)Cl(TPPTS)$_2$ · 6 H$_2$O

Variante A. In einem Schlenkrohr werden 1.84 g (1.0 mmol) RhCl(TPPTS)$_3$ · 9 H$_2$O in 40 ml stickstoffgesättigtem Wasser gelöst. Man erhält eine rote Lösung, in die über ein mit Fritte versehenes Gaseinleitungsrohr während 10 min Kohlenmonoxid eingeleitet wird. Die Lösung hellt bei Beginn des Einleitens deutlich auf. Nach Säulenchromatographie der rohen Substanz an Sephadex G-15 erhält man den Rhodium-Komplex analysenrein. Ausbeute: 1.22 g (95 %); gelbes Glas.

Charakterisierung

$^{31}$P-NMR (109.3 MHz, D$_2$O, 5 °C) : δ = 31.4 ppm (d) [$^1$J(Rh,P) = 129.8 Hz]
IR (cm$^{-1}$, KBr) : v(CO 1979 (sst), 1639 (st), v(SO) 1211 (Sch, sst), 1150 (sst), 1041 (sst), 789 (st)

| Elementaranalyse ($C_{37}H_{36}O_{25}ClNa_6P_2RhS_6$; 1411.27) | | | | | | | |
|---|---|---|---|---|---|---|---|
| Ber. | C 31.49 | H 2.57 | Cl 2.51 | O 28.34 | P 4.39 | Rh 7.29 | S 13.63 |
| Gef. | C 31.50 | H 2.70 | Cl 2.50 | O 29.94 | P 4.14 | Rh 7.30 | S 13.95 |

Variante B. 100 mg (0.25 mmol) Tetracarbonylbis($\mu$-chloro)dirhodium, [$(CO)_2RhCl]_2$, werden in 10 ml Toluol gelöst. Man fügt 570 mg (1.0 mmol) TPPTS in 10 ml Wasser hinzu und läßt das entstehende Zweiphasensystem 15 h bei Raumtemperatur rühren. Nach dieser Zeit hat sich die organische Phase entfärbt. Man trennt die Phasen und wäscht die organische Phase zweimal mit je 5 ml Wasser, die vereinigten wäßrigen Phasen zweimal mit je 5 ml Toluol. anschließend zieht man das Wasser im Vakuum einer Ölpumpe ab. Das Rohprodukt wird durch Säulenchromatographie an Sephadex G-15 gereinigt. Ausbeute: 590 mg (84 %); gelbes Pulver.

Charakterisierung

$^{31}$P-NMR  (109.3 MHz, $D_2O$, 5 °C): $\delta$ = 31.3 ppm (d) [$^1$J(Rh,P) = 128 Hz]
IR  (KBr, $cm^{-1}$): v(CO) = 1980 (sst)
  v(SO) = 1224 (Sch, sst), 1199 (sst), 1040 (sst)

| Elementaranalyse ($C_{37}H_{36}O_{25}ClNa_6P_2RhS_6$; 1411.27) | | | | | | | |
|---|---|---|---|---|---|---|---|
| Ber. | C 31.49 | H 2.57 | Cl 2.51 | O 28.34 | P 4.39 | Rh 7.29 | S 13.63 |
| Gef. | C 32.05 | H 2.53 | Cl 2.64 | O 28.26 | P 4.14 | Rh 7.30 | S 13.95 |

### Beispiel 14: Synthese von Rh(OH)(TPPTS)$_3$ · 9 $H_2O$

Eine Lösung von 260 mg (1.0 mmol) $RhCl_3$ · 3 $H_2O$ in 20 ml Wasser wird nach Zugabe von 5.68 g (10 mmol) TPPTS, gelöst in 10 ml Wasser, etwa 15 h gerührt. Die entstandene Lösung, die RhCl(TPPTS)$_3$, freies TPPTS, TPPOTS sowie geringe Mengen des zweikernigen Komplexes [Rh($\mu$-Cl)(TPPTS)$_2$Rh(TPPTS)$_2$]$_2$ enthält, läßt man mindestens 24 h bei Raumtemperatur stehen und arbeitet sie darauf durch Säulenchromatographie an Sephadex G-15 auf. Nach Abziehen des Lösungsmittels erhält man den chlorfreien Komplex Rh(OH)(H$_2$O)(TPPTS)$_3$ · 9 $H_2O$ in Ausbeuten von 70 bis 90 % als rotes Glas.

Charakterisierung

$^{31}$P-NMR  (109.3 MHz, $D_2O$, 21 °C) : $\delta$ = 35.2 ppm (dd) $^1$J(Rh,P$_A$) = 143.9 Hz $\delta$ = 53.48 ppm (dt);
  $^1$J(Rh,P$_B$) = 195.3 Hz
  $^2$J(P,P) = 40.9 Hz
IR  (KBr, $cm^{-1}$) : v(SO) 1220 (Sch, sst), 1196 (sst), 1038 (sst), 787 (m), 624 (m), 527 (s)

| Elementaranalyse ($C_{54}H_{55}Na_9O_{37}P_3RhS_9$; 1987.25) | | | | | | | |
|---|---|---|---|---|---|---|---|
| Ber. | C 33.63 | H 2.79 | Cl 0.0 | O 29.79 | P 4.68 | Rh 5.18 | S 14.51 |
| Gef. | C 32.59 | H 2.89 | Cl 0.0 | O 30.57 | P 4.33 | Rh 5.90 | S 14.10 |

Beispiel 15: Synthese von [Rh($\mu$-Cl)(CO)(TPPTS)]$_2$ · 6 H$_2$O

Zu einer Lösung von 190 mg (0.5 mmol) Bis[($\mu$-chloro)dicarbonylrhodium], [Rh($\mu$-Cl)(CO)$_2$]$_2$, in 10 ml Toluol gibt man 570 mg (1 mmol) TPPTS in 10 ml Wasser und läßt das entstehende Zweiphasengemisch 18 h bei Raumtemperatur rühren. Nach Trennung der Phasen wird die organische Phase zweimal mit je 5 ml Wasser gewaschen. Die vereinigten wäßrigen Phasen extrahiert man zweimal mit je 5 ml Toluol und filtriert. Aus dem Filtrat wird das Wasser im Vakuum einer Ölpumpe abgezogen. Die Reinigung erfolgt durch Säulenchromatographie an Sephadex G-15. Aus der ersten, orangefarbenen Zone isoliert man RhCl(CO)(TPPTS)$_2$ · 6 H$_2$O. Aus der nachfolgenden gelben Zone gewinnt man [Rh($\mu$-Cl)(CO)(TPPTS)]$_2$. Ausbeute: 330 mg (42 %); gelbes Pulver.

Charakterisierung

$^{31}$P-NMR    (109.3 MHz, D$_2$O, 5 °C) : $\delta$ = 48.2 ppm (d) [$^1$J(Rh,P) = 180 Hz] IR (KBr, cm$^{-1}$) : v(CO) = 1986 (sst), v(SO) = 1224 (Sch, sst), 1197 (sst), 1039 (sst)

| Elementaranalyse (C$_{38}$H$_{36}$Cl$_2$Na$_6$O$_{26}$P$_2$Rh$_2$S$_6$; 1577.65) | | | | | |
|---|---|---|---|---|---|
| Ber. | C 28.93 | H 2.30 | O 26.37 | P 3.93 | Rh 13.05 | S 12.19 |
| Gef. | C 29.90 | H 2.23 | O 27.02 | P 3.90 | Rh 13.15 | S 12.08 |

Beispiel 16: Synthese von Rh$_2$(TPPTS)$_2$[P(C$_6$H$_4$SO$_3$Na)$_2$(C$_6$H$_4$-m-SO$_3$)]$_2$ · 10 H$_2$O

Zu einer Lösung von 100 mg (0.25 mmol) Tetrakis($\eta^2$-ethylen)bis($\mu$-chloro)dirhodium, [Rh($\mu$-Cl)($\eta^2$-C$_2$H$_4$)$_2$]$_2$ in 10 ml Methylenchlorid gibt man 850 mg (1.5 mmol) TPPTS in 10 ml Wasser. Man läßt das Zweiphasensystem 30 min bei Raumtemperatur rühren, dabei entfärbt sich die organische Phase. Nach Trennung der Phasen wird die organische Phase zweimal mit je 5 ml Wasser extrahiert. Die vereinigten wäßrigen Phasen wäscht man zweimal mit je 5 ml Methylenchlorid und filtriert anschließend. Das Wasser wird im Vakuum einer Ölpumpe abgedampft. Das Rohprodukt wird durch Säulenchromatographie an Sephadex G-25 gereinigt. Nach einer grauen Bande folgt eine orangefarbene Zone, aus der die Rhodiumkomplexverbindung isoliert wird. Ausbeute: 320 mg (49 %); braunrotes Pulver.

Charakterisierung

$^{31}$P-NMR    (109.3 MHz, D$_2$O, 5 °C) : $\delta$ = 57.8 ppm (d) [$^1$J(Rh,P) = 181 Hz]
IR              (KBr, cm$^{-1}$) : v(SO) = 1224 (Sch, sst), 1199 (sst), 1139 (sst)

| Elementaranalyse (C$_{72}$H$_{68}$O$_{46}$P$_4$S$_{12}$Na$_{10}$Rh$_2$; 2613.63) | | | | | | |
|---|---|---|---|---|---|---|
| Ber. | C 33.09 | H 2.62 | Cl 0.00 | O 28.16 | P 4.74 | Rh 7.87 | S 14.72 |
| Gef. | C 33.04 | H 2.85 | Cl 0.00 | O 28.08 | P 4.48 | Rh 8.00 | S 14.16 |

Beispiel 17: Synthese von Rh$_2$($\mu$-Cl)$_2$($\eta^4$-C$_8$H$_{12}$)(TPPTS)$_2$ · 6 H$_2$O

Eine Lösung von 454 mg (0.8 mmol) TPPTS in 10 ml Wasser wird zu einer Lösung von 200 mg (0.4 mmol) [Rh($\mu$-Cl)($\eta^4$-C$_8$H$_{12}$)]$_2$ in 10 ml Methylenchlorid gegeben. Das erhaltene Zweisphasensystem rührt man 12 h bei Raumtemperatur. Dann isoliert man die wäßrige Phase, wäscht die organische Phase zweimal mit je 5 ml Wasser und die vereinigten wäßrigen Phasen zweimal mit je 5 ml Methylenchlorid. Zur Entfernung von Methylenchlorid-Resten wird die wäßrige Lösung auf 3/4 ihres ursprünglichen Volumens eingeengt. Die so erhaltene Lösung wird an einer Sephadex G-15-Säule chromatographiert. Ausbeute 510 mg (73 %); orangerotes Pulver.

Charakterisierung

$^{31}$P-NMR   (109.3 MHz, $D_2O$, 5 °C) : δ = 129.0 ppm (d) [$^1$J(Rh,P) = 146.2 Hz]
$^1$H-NMR   (400 MHz, $D_2O$, 23 °C) : δ = 2.14 ppm [br s, $CH_2$, 4H]; δ = 2.43 ppm [br s, $CH_2$, 4H]; δ = 4.54 ppm [CH, 4H]
; δ = 7.28-7.96 [m, $C_6H_4$, 24 H]
IR   ($cm^{-1}$ KBr) : 1633 (m), 1399 (st), v(SO) 1206 (Sch, sst), 1038 (st), 791 (st), 693 (m), 621 (m)

Beispiel 18: Synthese von $Rh_6(CO)_7(TPPTS)_9 \cdot 27 H_2O$

230 mg (0.07 mmol) $Rh_6(CO)_7[P(C_6H_5)_3]_9$ werden in 10 ml Chloroform gelöst. Unter Rühren gibt man 1.42 g (2.5 mmol) TPPTS in 10 ml Wasser zu, wobei sich die wäßrige Phase rasch gelb färbt. Zur Vervollständigung der Austauschreaktion läßt man noch 1 h rühren und trennt dann die Phasen. Die organische Phase wird zweimal mit je 5 ml Wasser gewaschen. Anschließend vereinigt man die wäßrigen Phasen und zieht das Wasser im Vakuum ab. Die Reinigung erfolgt durch Säulenchromatographie an Sephadex G-25. Ausbeute: 350 mg (78 %); gelbbraunes Pulver.

Charakterisierung

$^{31}$P-NMR   (109.3 MHz, $D_2O$, 5 °C) : δ = 30.5 ppm [$^1$J(Rh,P) = 130 Hz].
IR   ($cm^{-1}$, KBr) : v(SO) = 1225 (Sch, sst), 1200 (sst), 1039 (sst), 623 (sst); v(CO) = 1981 (st).

| Elementaranalyse ($C_{169}H_{162}Na_{27}O_{115}P_9Rh_6S_{27}$; 6415.61) | | | | |
|---|---|---|---|---|
| Ber. | C 31.64 | H 2.55 | O 28.68 | P 4.35 | S 13.49 |
| Gef. | C 31.96 | H 2.93 | O 28.83 | P 4.29 | S 13.69 |

Beispiel 19: Synthese von $Ir(NO)(TPPTS)_3 \cdot 9 H_2O$

Zu einer Lösung von 140 mg (0.17 mmol) Dichloronitrosylbis(triphenylphosphan)iridium, $IrCl_2NO [P(C_6H_5)_3]_2$ in 30 ml Methylenchlorid gibt man unter Rühren bei Raumtemperatur 850 mg (1.5 mmol) TPPTS in 10 ml Wasser. Bereits nach kurzer Zeit färbt sich die wäßrige Phase braun, während sich die organische Phase entfärbt. Zur Vervollständigung der Reaktion läßt man noch 1 h rühren und trennt dann die Phasen. Die organische Phase wird zweimal mit je 5 ml Wasser gewaschen. Die vereinigten wäßrigen Phasen extrahiert man zweimal mit je 5 ml Methylenchlorid und dampft anschließend das Wassr im Vakuum einer Ölpumpe ab. Das Rohprodukt wird durch Säulenchromatographie an Sephadex G-25 oder Fractogel TSK HW-40 F gereinigt. Ausbeute: 160 mg (45 %); braunes Pulver.

Charakterisierung

$^{31}$P-NMR   (109.3 MHz, $D_2O$, 5 °C) : δ = 16.7 ppm (s)
IR   (KBr, $cm^{-1}$) : v(SO) = 1225 (Sch, sst), 1198 (sst), 1039 (sst), 623 (st)

| Elementaranalyse ($C_{54}H_{54}IrNNa_9O_{37}P_3S_9$; 2089.59) | | | | | | |
|---|---|---|---|---|---|---|
| Ber. | C 31.04 | H 2.60 | Cl 0.0 | N 0.67 | O 28.33 | P 4.45 | S 13.81 |
| Gef. | C 29.42 | H 2.55 | Cl 0.0 | N 0.61 | O 27.90 | P 3.90 | S 13.41 |

Beispiel 20: Synthese von IrCl(CO)(TPPTS)$_3$ · 9 H$_2$O

160 mg (0.5 mmol) Ir(CO)$_3$Cl werden in 20 ml Toluol suspendiert. Unter starkem Rühren gibt man bei Raumtemperatur 1.14 g (2 mmol) TPPTS in 10 ml Wasser zu. Bereits nach kurzer Zeit färbt sich die wäßrige Phase gelb. Zur Vervollständigung der CO-Substitution läßt man noch 24 h bei Raumtemperatur rühren und trennt dann die Phasen. Die organische Phase wird zweimal mit je 5 ml Wasser gewaschen. Die vereinigten wäßrigen Phasen extrahiert man zweimal mit je 5 ml Toluol und filtriert anschließend. Nach Abziehen des Lösungsmittels im Vakuum einer Ölpumpe reinigt man das Rohprodukt durch Säulenchromatographie an Sephadex G-15. Ausbeute: 600 mg (57 %); orangegelbes Pulver.

Charakterisierung

$^{31}$P-NMR    (109.3 MHz, D$_2$O, 5 °C) : δ = -2.8 ppm (s)
IR          (KBr, cm$^{-1}$) : ν(CO) = 2005 (m), 1963 (m)
           ν(SO) = 1226 (Sch, sst), 1200 (sst), 1038 (sst), 623 (sst)

| Elementaranalyse (C$_{55}$H$_{54}$ClIrNa$_9$O$_{37}$P$_3$S$_9$; 2123.05) | | | | | |
|---|---|---|---|---|---|
| Ber. | C 31.11 | H 2.56 | Cl 1.68 | Ir 9.05 | O 27.88 | P 4.38 |
| Gef. | C 30.04 | H 2.54 | Cl 1.67 | Ir 8.76 | O 28.13 | P 3.99 |

Beispiel 21: Synthese von IrH(CO)(TPPTS)$_3$ · 9 H$_2$O

Zu 200 mg (0.2 mmol) Carbonylhydridotris(triphenylphosphan)iridium, IrH(CO)[P(C$_6$H$_5$)$_3$]$_3$, gelöst in 20 ml Toluol, gibt man 290 mg (0.5 mmol) TPPTS in 10 ml Wasser. Das resultierende Zweiphasengemisch läßt man 5 d unter Rückfluß sieden. Nach Abkühlen auf Raumtemperatur trennt man die Phasen und extrahiert die organische Phase mit 10 ml Wasser. Die vereinigten wäßrigen Phasen werden zweimal mit je 10 ml Toluol gewaschen und anschließend filtriert. Aus dem Filtrat wird das Wasser im Vakuum einer Ölpumpe abgedampft. Ausbeute: 310 mg (89 %, bezogen auf TPPTS); gelbes Pulver.

Charakterisierung

$^{31}$P-NMR    (161.9 MHz, D$_2$O, 5 °C) : δ = 19.2 ppm (s)
$^1$H-NMR    (400 MHz, D$_2$O, 5 °C) : δ = -10.69 ppm (q, IrH, 1H) [$^2$J(P,H) = 20.8 Hz]
           δ = 7.08 - 7.76 ppm (m, C$_6$H$_4$, 12H)
IR          (KBr, cm$^{-1}$) : ν(IrH) = 2128 (s),
           ν(CO) = 1927 (m),
           ν(SO) = 1222 (Sch, sst), 1196 (sst), 1038 (sst)

| Elementaranalyse (C$_{55}$H$_{55}$IrNa$_9$O$_{37}$P$_3$S$_9$; 2088.61) | | | | |
|---|---|---|---|---|
| Ber. | C 31.63 | H 2.65 | O 28.34 | P 4.45 |
| Gef. | C 31.65 | H 2.53 | O 28.23 | P 4.08 |

Beispiel 22: Synthese von $IrCl(\eta^4-C_8H_{12})(TPPTS)_2 \cdot 6\,H_2O$

Zu einer Lösung von 130 mg (0.2 mmol) $[(\mu-Cl)Ir(\eta^4-C_8H_{12})]_2$ in 10 ml Toluol gibt man unter Rühren bei Raumtemperatur 570 mg (1.0 mmol) TPPTS in 10 ml Wasser. Die organische Phase entfärbt sich spontan. Zur Vervollständigung der Reaktion läßt man noch 15 min rühren. Nach Trennung der Phasen wird das Wasser im Vakuum abgedampft. Die Reinigung des Rohproduktes erfolgt durch Säulenchromatographie an Sephadex G-15. Ausbeute: 770 mg (97 %); rote Kristalle.

Charakterisierung

$^{31}P$-NMR  (109.3 MHz, $D_2O$, 5 °C) : $\delta$ = 19.0 ppm (s).
$^1H$-NMR  (270 MHz, $D_2O$, 5 °C) : $\delta$ = 1.89 ppm [br, d, 4H, $^2J(H,H)$ = 7.8 Hz, $CH_2$], $\delta$ = 2.33 ppm [br, s, 4H, $CH_2$] ; $\delta$ = 4.32 ppm [br s, 4H, CH] ; $\delta$ = 7.48-7.96 ppm [m, 24H, $C_6H_4$]
IR  ($cm^{-1}$, KBr) : v(SO) = 1223 (Sch, sst), 1199 (sst), 1039 (sst), 623 (sst)

| Elementaranalyse ($C_{44}H_{48}ClIrNa_6O_{24}P_2S_6$; 1580.77) | | | | | |
|---|---|---|---|---|---|
| Ber. | C 33.43 | H 3.06 | Cl 2.24 | O 24.29 | P 3.92 | S 12.17 |
| Gef. | C 33.56 | H 2.99 | Cl 2.26 | O 23.75 | P 3.63 | S 12.87 |

Beispiel 23: Synthese von $Ni(TPPTS)_3 \cdot 9\,H_2O$

Variante A. 71 mg (0.3 mmol) $NiCl_2 \cdot 6\,H_2O$ und 0.85 g (1.5 mmol) TPPTS werden in einer Mischung aus 5 ml Wasser und 5 ml Ethanol gelöst. Bei -15 °C werden innerhalb 90 min 34 mg (0.9 mmol) $Na[BH_4]$, gelöst in 5 ml Wasser und 5 ml Ethanol, zugetropft. Dabei färbt sich die Reaktionslösung rot und schließlich rotbraun. Nach Beendigung der $Na[BH_4]$-Zugabe läßt man innerhalb 3 h die Temperatur der Lösung auf Raumtemperatur ansteigen und zieht das Lösungsmittel im Vakuum ab. Die Reinigung des Rohproduktes erfolgt durch Säulenchromatographie an Sephadex G-15 unter Kühlung der Säule auf 0 °C. Ausbeute: 0.53 g (92 %); rotbraunes Pulver.

Charakterisierung

$^{31}P$-NMR  (109.3 MHz, $D_2O/C_2H_5OH$ 1:1, -30 °C) : $\delta$ = 22.7 ppm (s).
Die chemische Verschiebung von $Ni(TPP)_3$ beträgt $\delta$ = 23 ppm [C. A. Tolman, W. C. Seidel und D. H. Gerlach, J. Am. Chem. Soc. 94 (1972) 2669].
IR  (KBr, $cm^{-1}$) : v(SO) = 1222 (Sch, sst), 1192 (sst), 1039 (sst), 622 (sst)

| Elementaranalyse ($C_{54}H_{54}Na_9NiO_{36}P_3S_9$: 1926.07) | | | | |
|---|---|---|---|---|
| Ber. | C 33.67 | H 2.83 | P 4.82 | Ni 3.05 | P 4.82 |
| Gef. | C 33.74 | H 2.87 | P 4.69 | Ni 3.00 | P 4.79 |

Variante B. Zu einer Lösung von 100 mg (0.36 mmol) $Bis(\eta^4-1.5$-cyclooctadien)nickel $Ni(\eta^4-C_8H_{12})_2$ in 10 ml Toluol gibt man unter starkem Rühren 830 mg (1.45 mmol) TPPTS in 10 ml Wasser; dabei färbt sich die wäßrige Phase rasch rotbraun. Zur Vervollständigung der Austauschreaktion rührt man das Zweiphasensystem weitere 8 h bei Raumtemperatur. Danach hat sich die organische Phase entfärbt. Die Phasen werden getrennt. Man wäscht die organische Phase zweimal mit je 5 ml Wasser und extrahiert die vereinigten wäßrigen Phasen zweimal mit je 5 ml Toluol. Anschließend wird das Wasser im Vakuum abgedampft. Der Rückstand wird durch Säulenchromatographie an Sephadex G-15 bei 0 °C gereinigt (Glassäule, 1 = 60 cm, d = 1 cm, Kühlung mit Kyromat Julabo F 40, ein Umwälzkryomat der Fa. Julabo). Nach einer grünen Zone, die Zersetzungsprodukte enthält, folgt eine breite, rotbraune Zone, aus der der Nickelkomplex isoliert wird. Ausbeute: 380 mg (55 %); rotbraunes Pulver.

Charakterisierung

$^{31}$P-NMR (109.3 MHZ, $D_2O/C_2H_5OH$ 1:1, -30 °C) : $\delta$ = 22.7 ppm (s).
Die chemische Verschiebung von Ni(TPP)$_3$ beträgt $\delta$ = 23 ppm [C. A. Tolman, W. C. Seidel und D. H. Gerlach, J. Am. Chem. Soc. 94 (1972) 2669].
IR (KBr, cm$^{-1}$) : v(SO) = 1222 (Sch, sst), 1192 (sst), 1039 (sst), 622 (sst).

Variante C. Zu einer Lösung von 330 mg (0.3 mmol) Tetrakis(triphenylphosphan)nickel, Ni[P(C$_6$H$_5$)$_3$]$_4$, in 10 ml Toluol gibt man unter Rühren bei Raumtemperatur 680 mg (1.2 mmol) TPPTS in 10 ml Wasser. Die wäßrige Phase färbt sich rasch rotbraun. Zur Vervollständigung der Austauschreaktion läßt man noch 15 h rühren und trennt dann die Phasen. Die organische Phase wird zweimal mit je 5 ml Wasser gewaschen, die vereinigten wäßrigen Phasen werden zweimal mit je 5 ml Toluol extrahiert. Anschließend wird das Wasser im Vakuum einer Ölpumpe abgezogen. Das Rohprodukt wird durch Säulenchromatographie an Sephadex G-15 gereinigt. Ausbeute: 350 mg (61 %); rotbraunes Pulver.

Charakterisierung

$^{31}$P-NMR (109.3 MHz, $D_2O/C_2H_5OH$, -30 °C) : $\delta$ = 22.7 ppm (s)
Die chemische Verschiebung von Ni(TPP)$_3$ beträgt $\delta$ = 23 ppm [C. A. Tolman, W. C. Seidel und D. H. Gerlach, J. Am. Chem. Soc. 94 (1972) 2669].
IR (KBr, cm$^{-1}$) : v(SO) = 1222 (Sch, sst), 1192 (sst), 1039 (sst), 622 (sst)

| Elementaranalyse ($C_{54}H_{54}Na_9NiO_{36}P_3S_9$; 1926.07) | | | | | |
|------|-----------|---------|----------|---------|----------|
| Ber. | C 33.67 | H 2.83 | Ni 3.05 | P 4.82 | O 29.90 |
| Gef. | C 33.74 | H 2.87 | Ni 3.49 | P 4.69 | O 30.61 |

Beispiel 24: Synthese von Ni(CO)$_2$(TPPTS)$_2$ · 6 H$_2$O

Variante A.

170 mg (1 mmol) Ni(CO)$_4$ werden in 10 ml Toluol gelöst und mit 2.84 g (5 mmol) TPPTS, gelöst in 10 ml Wasser, versetzt. Das resultierende Zweiphasengemisch läßt man 18 h bei 25 °C rühren. Man wäscht die wäßrige Phase mit 10 ml Toluol und zieht anschließend das Wasser im Vakuum ab. Das Rohprodukt wird durch Gelchromatographie an Sephadex G-15 gereinigt (Säule 1 = 100 cm, $\varnothing$ = 24 mm). Ausbeute 0.83 g (61 %); gelbes Pulver.

Charakterisierung

$^{31}$P-NMR (109.3 MHz, D$_2$O, 5 °C) : $\delta$ = 34.9 ppm (Singulett)
IR (cm$^{-1}$, KBr) : v(CO) = 1944(st), 2008(st) ; v(SO) = 1222(Sch,sst), 1196(sst), 1040(sst), 624(st)

| Elementaranalyse ($C_{38}H_{36}Na_6NiP_2O_{26}S_6$; 1358.63) | | | | | |
|------|-----------|---------|----------|----------|---------|
| Ber. | C 33.57 | H 2.67 | Ni 4.32 | O 30.60 | P 4.56 |
| Gef. | C 33.52 | H 2.52 | Ni 4.52 | O 29.23 | P 4.25 |

Variante B. Durch eine Lösung von 190 mg (0.1 mmol) Ni(TPPTS)$_3$ · 9 H$_2$O in 10 ml Wasser leitet man bei Raumtemperatur 15 min lang einen CO-Gasstrom; die ursprünglich tiefrotbraune Lösung färbt sich rasch gelb. Anschließend läßt man noch 15 min rühren und zieht dann das Lösungsmittel im Vakuum einer Ölpumpe ab. Der Rückstand wird durch Säulenchromatographie an Sephadex G-15 gereinigt. Ausbeute: 110 mg (81 %); gelbes Pulver.

Charakterisierung

$^{31}$P-NMR  (109.3 MHz, $D_2O/C_2H5OH$ 1:1, -30 °C) : $\delta$ = 22.7 ppm (s).
Die chemische Verschiebung von Ni(TPP)3 beträgt $\delta$ = 23 ppm [C. A. Tolman, W. C. Seidel und D. H. Gerlach, J. Am. Chem. Soc. 94 (1972) 2669].

IR  (KBr, cm$^{-1}$) : v(SO) = 1222 (Sch, sst), 1192 (sst), 1039 (sst), 622 (sst)

| Elementaranalyse ($C_{38}H_{36}Na_6NiP_2O_{26}S_6$; 1358.63) | | | | | |
|---|---|---|---|---|---|
| Ber. | C 33.57 | H 2.67 | Ni 4.32 | O 30.60 | P 4.56 |
| Gef. | C 33.52 | H 2.52 | Ni 4.52 | O 29.23 | P 4.25 |

Beispiel 25: Synthese von Ni(PF$_3$)$_2$(TPPTS)$_2$ · 6 H$_2$O

Variante A. 0.21 g (0.5 mmol) Ni(PF$_3$)$_4$ werden in 20 ml Tetrahydrofuran gelöst. Zu der Lösung gibt man 0.85 g (1.5 mmol) TPPTS in 10 ml Wasser und erhitzt 4 h zum Sieden, die wäßrige Phase hat sich dann gelb gefärbt. Nach Abkühlen auf Raumtemperatur trennt man die Phasen, wäscht die Wasser-Phase zweimal mit je 5 ml Toluol und zieht dann das Lösungsmittel im Vakuum ab. Die Reinigung des Rohproduktes erfolgt durch Säulenchromatographie an Sephadex G-15. Ausbeute: 0.56 g (76 %); gelbes Pulver.

Variante B. 0.13 g (0.3 mmol) Ni(PF$_3$)$_4$ werden in 10 ml Tetrahydrofuran und 10 ml Toluol gelöst. Dazu gibt man 0.51 g (0.9 mmol) TPPTS in 10 ml Wasser und läßt das resultierende Zweiphasen-Gemisch 3 h bei 25 °C rühren. Nach dieser Zeit hat sich die wäßrige Phase gelb gefärbt. Die Phasen werden getrennt, die wäßrige Phase wird zweimal mit je 5 ml Toluol gewaschen und zur Trockne eingedampft. Die Reinigung des Rohproduktes erfolgt durch Säulenchromatographie Sephadex G-15. Ausbeute: 0.35 g (78 %); gelbes Pulver.

Charakterisierung

$^{31}$P-NMR  (109.3 MHz, $D_2O$/EtOH 1:1, -30 °C): $\delta$ = 45.8 ppm.

| Elementaranalyse ($C_{36}H_{36}F_6Na_6NiO_{24}P_4S_6$; 1479.55) | | | | | | |
|---|---|---|---|---|---|---|
| Ber. | C 29.23 | H 2.45 | F 7.71 | Ni 3.97 | O 25.95 | P 8.37 | S 13.00 |
| Gef. | C 29.10 | H 2.50 | F 7.60 | Ni 3.89 | O 26.15 | P 8.50 | S 13.50 |

Beispiel 26: Synthese von Pd(TPPTS)$_3$ · 9 H$_2$O

Variante A. Zu einer Lösung von 0.46 g (0.4 mmol) Tetrakis(triphenylphosphan)palladium, Pd[P(C$_6$H$_5$)$_3$]$_4$, in 40 ml Toluol gibt man bei 25 °C 2.27 g (4 mmol) TPPTS in 20 ml Wasser. Die organische Phase entfärbt sich schnell. Zur Vervollständigung der Reaktion läßt man noch 15 min rühren. Darauf trennt man die Phasen und wäscht die Toluol-Phase zweimal mit je 5 ml Wasser. Die vereinigten wäßrigen Phasen werden filtriert, anschließend dampft man das Wasser im Vakuum ab. Der Rückstand wird durch Säulenchromatographie an Sephadex G-25 gereinigt. Ausbeute: 0.41 g (52 %); braunes Pulver.

Variante B. Zu einer Lösung von 0.32 g (1 mmol) Dikaliumtetrachloropalladat(II), $K_2[PdCl_4]$, in 10 ml Wasser werden bei 25 °C unter Rühren 2.84 g (5 mmol) TPPTS in 10 ml Wasser gegeben, wobei sich die Reaktionslösung braun färbt. Dann werden während 30 min 170 mg (4.5 mmol) Natriumtetrahydridoborat, $Na[BH_4]$, in 5 ml Wasser zugetropft. Man läßt noch 90 min rühren und zieht darauf das Wasser im Vakuum ab. Der Rückstand wird zweimal mit je 5 ml Ethanol gewaschen und anschließend durch Säulenchromatographe über Sephadex G-25 gereinigt. Ausbeute: 1.62 g (82 %); braunes Pulver.

Charakterisierung

$^{31}$P-NMR  (109.3 MHz, $D_2O$, 5 °C: $\delta$ = 22.6 ppm (s).
IR       ($cm^{-1}$, KBr): v(SO) = 1225 (Sch, sst), 1200 (sst), 1039 (sst), 622 (sst).

| Elementaranalyse ($C_{54}H_{54}Na_9O_{36}P_3PdS_9$; 1973.77) | | | | | | |
|------|-----------|---------|----------|---------|----------|----------|
| Ber. | C 32.86 | H 2.76 | O 29.18 | P 4.71 | Pd 5.39 | S 14.62 |
| Gef. | C 32.35 | H 2.70 | O 29.95 | P 4.87 | Pd 5.30 | S 15.27 |

Beispiel 27: Synthese von Pt(TPPTS)$_4$ · 12 $H_2O$

Variante A. 0.78 g (0.7 mmol) Tetrakis(triphenylphosphan)platin, $Pt[P(C_6H_5)_3]_4$, werden in 80 ml Toluol gelöst. Zu der Lösung gibt man unter Rühren 3.18 g (5.6 mmol) TPPTS in 30 ml Wasser. Dabei entfärbt sich die organische Phase schnell. Zur Vervollständigung der Reaktion rührt man noch 15 min bei Raumtemperatur. Nach Trennung der Phasen wird die organische Phase zweimal mit je 10 ml Wasser gewaschen. Die vereinigten wäßrigen Lösungen werden filtriert, das Wasser wird im Vakuum abgezogen. Man reinigt die Substanz durch Säulenchromatographie an Sephadex G-25. Ausbeute: 750 mg (40 %); gelborange Kristalle.

Variante B. Eine Lösung von 2.84 g (5 mmol) TPPTS in einem Gemisch aus 10 ml Wasser und 20 ml Ethanol wird auf 70 °C erwärmt. Nach Zugabe von 80 mg (2 mmol) Natriumhydroxid werden innerhalb 1 h insgesamt 0.42 g (1 mmol) Dikalium-tetrachloroplatinat(II), $K_2[PtCl_4]$, in 5 ml Wasser zugetropft. Die Lösung färbt sich orangegelb. Man läßt noch 2 h rühren und zieht dann das Lösungsmittel im Vakuum ab. Der Rückstand wird durch Säulenchromatographie an Sephadex G-25 gereinigt. Ausbeute: 1.21 g (45 %); gelborange Kristalle.

Variante C. 200 mg (0.24 mmol) des Peroxo-Komplexes Pt($\eta^2$-$O_2$) $[P(C_6H_5)_3]_2$ · $C_6H_6$ werden in 10 ml Methylenchlorid gelöst. Man fügt 1.71 g (3 mmol) TPPTS in 10 ml Wasser hinzu und läßt das resultierende Zweiphasensystem 5 h bei 25 °C rühren. Nach Trennung der Phasen wird die organische Phase mit 10 ml Wasser gewaschen. Die vereinigten wäßrigen Phasen wäscht man mit 10 ml Methylenchlorid, darauf zieht man Wasser im Vakuum ab und reinigt das Rohprodukt durch Säulenchromatographie an Sephadex G-25. Ausbeute: 240 mg (37 %); gelbe Kristalle.

Charakterisierung

$^{31}$P-NMR  (109.3 MHz, $D_2O$, 5 °C) : $\delta$ = 22.2 ppm (dt) [$^1$J(Pt,P) = 2853 Hz, $^3$J(P,P) = 19.8 Hz] ; $\delta$ = 24.1 ppm (m) [$^1$J(Pt-P) = 2210 Hz].
IR       (KBr, $cm^{-1}$) : v(SO) = 1226 (Sch, sst), 1201 (sst), 1039 (sst), 622 (sst)

| Elementaranalyse ($C_{72}H_{60}Na_{12}O_{48}P_4PtS_{12}$; 2684.94) | | | | | | |
|------|-----------|---------|----------|---------|----------|----------|
| Ber. | C 32.21 | H 2.70 | O 28.60 | P 4.61 | Pt 7.27 | S 14.33 |
| Gef. | C 32.21 | H 2.58 | O 27.62 | P 4.61 | Pt 7.03 | S 14.86 |

Beispiel 28: Synthese von PtCl$_2$(TPPTS)$_2$ · 6 H$_2$O

Eine Lösung von 0.21 g (0.5 mmol) Dikalium-tetrachloroplatinat(II), K$_2$[PtCl$_4$], in 5 ml Wasser, wird bei 25 °C so langsam zu 0.57 g (1 mmol) TPPTS in 10 ml Wasser getropft, daß immer Entfärbung eintritt. Man läßt 20 h rühren und zieht anschließend das Wasser im Vakuum ab. Das Produkt enthält noch restliches Kaliumchlorid, das gelchromatographisch nicht abgetrennt werden kann, da sich der Platin-Komplex an den gängigen Trägermaterialien zersetzt. Ausbeute: 0.69 g (91 %); gelbliches Pulver.

Charakterisierung

$^{31}$P-NMR   (109.3 MHz, D$_2$O, 5 °C) : δ = 13.9 ppm (t) [$^1$J(Pt,P) = 3727 Hz]
IR           (cm$^{-1}$, KBr): ν(SO) = 1226 (Sch, sst), 1201 (sst), 1039 (sst), 623 (sst) ; ν(Pt-Cl) = 313 (s)

In den folgenden Beispielen 34 bis 56 wird die Verwendung der neuen Verbindungen als Katalysatoren beschrieben.

Beispiel 34: Katalytische Hydrierung von Cyclohexen in Gegenwart von Co$_2$(CO)$_6$(TPPTS)$_2$ · 6 H$_2$O als Katalysator

In einem 100 ml-Laborautoklaven werden unter Argon 31 mg (0.02 mmol) Co$_2$(CO)$_6$(TPPTS)$_2$ · 6 H$_2$O, gelöst in 2 ml Wasser, sowie 820 mg (10 mmol) frisch destilliertes Cyclohexen vorgegeben. Man läßt 20 h bei 3,0 MPa Wasserstoff-Druck und 20 °C reagieren und trennt darauf die Phasen. Die organische Phase besteht nach GC/MS-Analyse aus 7 % Cyclohexan und 93 % Cyclohexen.

Beispiel 35: Selektive Hydrierung von 1-Decen in Gegenwart von Co$_2$(CO)$_6$(TPPTS)$_2$ · 6 H$_2$O unter Hydroformylierungsbedingungen als Katalysator

In einem 15 min mit Stickstoff gespülten 50 ml-Laborautoklaven werden eine Lösung von 31 mg (0.02 mmol) Co$_2$(CO)$_6$(TPPTS)$_2$ · 6 H$_2$O in 2 ml Wasser und 0.70 g (5 mmol) 1-Decen vorgelegt. Anschließend läßt man 16 h bei 100 °C und einem Druck von 7.0 MPa H$_2$/CO (1:1) reagieren. Nach Abkühlen auf 25 °C wird die organische Phase mit 2 ml Methylenchlorid extrahiert. Die GC/MS-Analyse ergibt 76 % n-Decan und 24 % unverändertes 1-Decen. Ein entsprechender Aldehyd konnte nicht nachgewiesen werden.

Beispiel 36: Katalytische Hydrierung von Cyclohexen und cis-Cyclooocten in Gegenwart von Rh(NO)(TPPTS)$_3$ · 9 H$_2$O als Katalysator

a) Hydrierung von Cyclohexen ohne Lösungsmittel: In einer Hydrierapparatur nach Marhan werden 20 mg Rh(NO)(TPPTS)$_3$ · 9 H$_2$O (0.01 mmol) in 1.5 ml destilliertem Wasser vorgelegt. Zu dieser Lösung gibt man 330 mg (4 mmol) Cyclohexen, spült die Apparatur mit H$_2$ und läßt bei 25 °C und 0.1 MPa H$_2$-Druck 92 h reagieren. Die organische Phase wird mit 3 ml Methylenchlorid aufgenommen und über waserfreiem Natriumsulfat getrocknet. Das Produkt besteht nach GC/MS-Analyse aus 63.3 % Cyclohexan und 36.7 % Cyclohexen.

b) Hydrierung von Cyclohexen in Lösung: In einer Hydrierapparatur nach Marhan werden 40 mg (0.02 mmol) Rh(NO)(TPPTS)$_3$ · 9 H$_2$O in einem Gemisch aus 3 ml Wasser und 3 ml Isopropanol vorgelegt. Dazu gibt man 410 mg (5 mmol) Cyclohexen, spült die Apparatur kurz mit H$_2$ und läßt bei 25 °C und 0.1 MPa H$_2$-Druck 120 h reagieren. Die organische Phase wird mit 3 ml Methylenchlorid aufgenommen und über wasserfreiem Na$_2$SO$_4$ getrocknet. Das Produkt besteht nach GC/MS-Analyse aus 47 % Cyclohexan und 53 % Cyclohexen.

c) Hydrierung von Cyclooocten unter Normaldruck: In einer Hydrierapparatur nach Marhan werden unter H$_2$-Atmosphäre (0.1 MPa) 100 mg (0.05 mmol) Rh(NO)(TPPTS)$_3$ · 9 H$_2$O in 5 ml Wasser vorgelegt. Dazu gibt man 2.20 g (20 mmol) frisch destilliertes cis-Cyclooocten und rührt das resultierende Zweiphasen-Gemisch 43 h bei 25 °C. Die GC/MS-Analyse der organischen Phase ergibt ein Cyclooctan/Cyclooocten-Verhältnis von 30 : 70. Nach 150 h Versuchsdurchführung beträgt das Cyclooctan/Cyclooocten-Verhältnis 98 : 2.

Beispiel 37: Katalytische Hydrierung von cis-Cyclooocten mit IrCl(η-1,5-C$_8$H$_{12}$)(TPPTS)$_2$ · 6 H$_2$O als Katalysator

In einen 50 ml-Laborautoklaven werden 2.75 g (25 mmol) cis-Cyclooocten und 79 mg (0.05 mmol) (η$^4$-1,5-C$_8$H$_{12}$) IrCl(TPPTS)$_2$ · 6 H$_2$O in 3 ml Wasser gegeben. Nach zweimaligem Spülen des Autoklaven mit Wasserstoff preßt man H$_2$ bis zu einem Druck von 2,5 MPa auf und erhitzt dann 20 h auf 100 °C. Der Druck steigt auf 3 MPa. Nach Abkühlen auf Raumtemperatur trennt man die Phasen. Die GC/MS-Analyse der organischen Phase ergibt, daß im Reaktionsprodukt 64 % Cyclooctan und 36 % cis-Cycloocten vorhanden sind.

Beispiel 38: Katalytische Reduktion von Nitrobenzol mit Kohlenmonoxid in Gegenwart von Fe-TPPTS-Komplexverbindungen als Katalysator

a) 470 mg (1.29 mmol) $Fe_2(CO)_9$ werden mit einer Lösung von 587 mg (1.29 mmol) TPPTS in 50 ml Wasser $t_1$ [min] gekocht. Darauf setzt man 10 ml Nitrobenzol mit 3 ml Ethanol zu und kocht weitere $t_2$ [min] (vgl. Tabelle 1) unter Rückfluß und CO-Einleitung (0.1 MPa). Nach dem Abkühlen werden die Phasen getrennt. Die Nitrobenzol-Phase wird über ein dichtes Filter von feinverteiltem Eisenhydroxid befreit und dann mit 1 %iger Salzsäure ausgeschüttelt. Dieser Extrakt wird eingedampft und die Ausbeute an Anilin bestimmt. Zusätzlich entnimmt man dem Nitrobenzol vor dem Ausschütteln mit Salzsäure eine Probe für die GC/MS-Analyse. Die Ergebnisse sind in Tabelle 1 zusammengefaßt.

Aus diesen Ansätzen läßt sich als katalytisch wirksame Verbindung der rote Komplex $Fe_4(CO)_{11}(TPPTS)$ isolieren, der gelchromatographisch an Sephadex G-15 zu reinigen ist. Dazu verfährt man folgendermaßen:

Das wäßrige Filtrat wird im Vakuum auf 30 ml eingeengt. Zur Isolierung des katalytisch aktiven Komplexes ordnet man 40 g Sephadex G-15 auf einer Glasfritte von etwa 4 cm Durchmesser in einer 25-30 cm hohen Schicht an. Im schnellen Strom gibt man das Filtrat auf und saugt zwei gelbe Fraktionen ab; diese enthalten die Komplexe $Fe(CO)_4(TPPTS)$ und $Fe(CO)_3(TPPTS)_2$ (vgl. Beispiel 2). Nun eluiert man bei einer Flußgeschwindigkeit von 1 Tropfen/sec mit reinem Wasser solange eine braune Zone, bis die zuletzt übrigbleibende tiefrote, äußerst langsam wandernde Zone das Säulenende erreicht hat. Mit Wasser/Ethanol (Verhältnis 3:1, insgesamt 50 ml; dann Verhältnis 1:1) extrahiert man einen roten, intensiv färbenden Komplex, bis das Gel nur mehr leicht rosa gefärbt ist; man benötigt hierfür mindestens 750 ml Elutionsmittel. Nach Abziehen des Lösungsmittels im Vakuum verbleiben 110 mg der neuen Verbindung (2 Gew.-%, bezogen auf eingesetztes $Fe_2(CO)_9$).

Charakterisierung

$^{31}$P-NMR  (109.3 MHz, $D_2O$ + 5 °C): δ = 84.81 ppm.
IR    [KBr, $cm^{-1}$]: 2052 m, 2002 s, Sch, 1963 s, 1900-1835 (zahlreiche Schultern).

| Elementaranalyse ($C_{29}H_{18}Fe_4Na_3O_{23}PS_3$; 1153.92) | | | | |
|---|---|---|---|---|
| Ber. | C 30.19 | H 1.57 | Fe 19.36 | P 2.68 |
| Gef. | C 30.00 | H 1.40 | Fe 19.00 | P 2.40 |

Tabelle 1

| Hydrierung von Nitrobenzol | | | | |
|---|---|---|---|---|
| $t_1$ (min) | $t_2$ (min) | Druck (MPa) | Molverhältnis*) (GC/MS-Bestimmung) | Anilin · HCl (mg) |
| 15 | 120 | 0,1 | 1/134 (= 0.75 %) | 89 |
| 20 | 120 | 0,1 | 1/100 (= 1 %) | 120 |
| 30 | 120 | 0,1 | 1/108 (= 0.93 %) | 112 |
| 60 | 120 | 0,1 | 1/23 (= 4.4 %) | 380 |

*) Anilin/Nitrobenzol

b) 45 mg (0.13 mmol) $Fe_3(CO)_{12}$ werden mit 175 mg (0.28 mmol) TPPTS in 7 ml Wasser und 3 ml Ethanol 1 h unter Rückfluß gekocht. Dabei scheidet sich ein schwarzes Pulver ab. Die gelbe, filtrierte Lösung wird mit 3 ml 10 %iger KOH und 10 ml Nitrobenzol versetzt. Unter CO-Einleitung kocht man weitere 2 h unter Rückfluß. Nach Phasentrennung wird die Nitrobenzol-Phase wie unter a) behandelt.

Analyse: GC/MS, Anilin/Nitrobenzol 1/20; Dünnschichtchromatogramm/(Kieselgelplatte, Toluol/Methanol 10/1 als Laufmittel, Platte nicht gesättigt): $R_f$ = 0.49 [Anilin], $R_f$ = 0.87 [Nitrobenzol], $R_f$ = 0.30 [unbekannt].

Beispiel 39: Katalytische Reduktion von Nitrobenzol mit CO in Gegenwart von $Co_2(CO)_6(TPPTS)_2$ · 6 $H_2O$ als Katalysator

In einem 100 ml-Laborautoklaven, der vorher 15 min mit Stickstoff gespült war, werden 62 mg (0.04 mmol) $Co_2(CO)_6(TPPTS)_2$ · 6 $H_2O$ in 5 ml Wasser und 490 mg (4 mmol) Nitrobenzol vorgegeben.

(a) Man läßt 12 h bei 100 °C und 4,0 MPa CO-Druck rühren und extrahiert dann die organische Phase mit 5 ml Methylenchlorid. Die GC/MS-Analyse ergibt 4 % Anilin und 96 % Nitrobenzol.
(b) Man verfährt wie unter (a), rührt jedoch 40 h bei 140 °C und 2,5 MPa CO-Druck. Ergebnis (GC/MS-Analyse): 16.4 % Anilin, 83.6 % Nitrobenzol.
(c) Man verfährt wie unter (a), rührt jedoch 40 h bei 140 °C und 5,5 MPa CO-Druck. Ergebnis (GC/MS-Analyse): 38.4 % Anilin, 61.6 % Nitrobenzol.

Beispiel 40: Hydrocarbonylierung von Ethylen mit $Co_2(CO)_6(TPPTS)_2$ · 6 $H_2O$ als Katalysator

In einem 50 ml-Laborautoklaven werden unter Stickstoffatmosphäre 153 mg (0.1 mmol) $Co_2(CO)_6(TPPTS)_2$ · 6 $H_2O$ in 5 ml Wasser vorgelegt. Sodann preßt man Ethylen bis zu einem Druck von 0,5 MPa (etwa 10 mmol) und CO bis zu einem Druck von 4 MPa auf. Man erhitzt auf 145 °C und läßt 40 h bei dieser Temperatur reagieren. Nach Abkühlen auf Raumtemperatur gibt man 2 ml Methylenchlorid zu und untersucht die organische Phase durch GC/MS-Analyse; neben Methylenchlorid läßt sich nur noch Diethylketon [$v(CO)$ = 1729 cm$^{-1}$] nachweisen. Der Umsatz beträgt etwa 10 %.

Beispiel 41: Hydroformylierung von 1-Hexen in Gegenwart von $Co_2(CO)_6(TPPTS)_2$ · 6 $H_2O$ als Katalysator

1. In einem 50 ml-Laborautoklaven werden unter Stickstoffatmosphäre 62 mg (0.04 mmol) $Co_2(CO)_6(TPPTS)_2$ · 6 $H_2O$ und 0.45 g (0.08 mmol) TPPTS in 3 ml Wasser vorgelegt. Dazu gibt man 1.62 g (20 mmol) 1-Hexen und preßt CO/$H_2$ bis zu einem Druck von 7 MPa auf. Man erhitzt auf 110 °C und läßt 18 h bei dieser Temperatur reagieren. Nach Abkühlen auf Raumtemperatur trennt man die Phasen. Die GC/MS-Analyse der organischen Phase ergibt, daß der Umsatz von 1-Hexen zu Heptanal(1) 36 % beträgt, bei einem n/i-Verhältnis von 3.4 : 1.
2. Die wäßrige Phase wird erneut mit 1.62 g (20 mmol) 1-Hexen versetzt. Die Hydroformylierung wird unter den Bedingungen von 1. wiederholt. Die GC/MS-Analyse der organischen Phase ergibt, daß der Umsatz von 1-Hexen zu Heptanal(1) 35 % beträgt mit einem n/i-Verhältnis von 2.1 : 1.
3. In einem 50 ml-Laborautoklaven werden unter Stickstoffatmosphäre 153 mg (0.01 mmol) $Co_2(CO)_6(TPPTS)_2$ · 6 $H_2O$ in 5 ml Wasser vorgelegt. Dazu gibt man 0.84 g (1 mmol) 1-Hexen und preßt CO bis zu einem Druck von 5,5 MPa auf. Das Zweiphasengemisch wird auf 170 °C erhitzt. Nach 20 h Reaktion bei dieser Temperatur läßt man auf Raumtemperatur abkühlen und trennt die Phasen. Die GC/MS-Analyse der organischen Phase ergibt, daß 1-Hexen vollständig zu Heptanal(1) umgesetzt wird bei einem n/i-Verhältnis von 1 : 1.

Beispiel 42: Hydroformylierung von Ethylen mit $RhCl(CO)(TPPTS)_2$ · 6 $H_2O$ als Katalysator unter CO-Atmosphäre

In einem 50 ml-Laborautoklaven werden unter Stickstoffatmosphäre 141 mg (0.1 mmol) $RhCl(CO)(TPPTS)_2$ · 6 $H_2O$ gelöst in 3 ml Wasser vorgelegt. Sodann preßt man Ethylen bis zu einem Druck von 0,5 MPa (etwa 10 mmol) und anschließend CO bis zu einem Druck von 3 MPa auf. Man erhitzt auf 120 °C und läßt 22 h bei dieser Temperatur reagieren. Nach Abkühlen auf 0 °C isoliert man die organische Phase. Ihre GC/MS- und GC/IR-Analyse ergibt, daß sich 0.65 g (etwa 20 %) Propanal [$v(CO)$ = 1743 cm$^{-1}$] gebildet haben.

Beispiel 43: Hydroformylierung von 1-Hexen mit $RhCl(CO)(TPPTS)_2$ · 6 $H_2O$ als Katalysator unter CO-Atmosphäre

In einem 50 ml-Laborautoklaven werden unter Stickstoffatmosphäre 70 mg (0.05 mmol) $RhCl(CO)(TPPTS)_2$ · 6 $H_2O$ in 3 ml Wasser vorgelegt. Dazu gibt man 0.84 g (10 mmol) 1-Hexen und preßt CO bis zu einem Druck von 4 MPa auf. Das Zweiphasengemisch wird auf 100 °C erhitzt. Nach 18 h Reaktion bei dieser Temperatur läßt man auf Raumtemperatur abkühlen und isoliert die organische Phase. Ihre GC/MS-Analyse ergibt, daß der Umsatz von 1-Hexen zu Heptanal(1) 92 % beträgt bei einem n/i-Verhältnis von 76 : 24.

Beispiel 44: Hydroformylierung von 1-Hexen in Gegenwart von $Rh_6(CO)_7(TPPTS)_9 \cdot 27\,H_2O$ als Katalysator

Ein 100 ml-Laborautoklav wird unter Stickstoffatmosphäre mit 65 mg (0.01 mmol) $Rh_6(CO)_7(TPPTS)_9 \cdot 27\,H_2O$ und 0.17 g (0.3 mmol) TPPTS in 3 ml Wasser beschickt. Nach Zugabe von 1.62 g (20 mmol) 1-Hexen preßt man $CO/H_2$ bis zu einem Druck von 5 MPa auf und erhitzt das Zweiphasensystem unter kräftigem Rühren 18 h auf 105 °C, wobei der Druck auf 5,7 MPa steigt. Nach Beendigung der Reaktion werden die Phasen getrennt. Die organische Phase wird durch GC/MS-Analyse untersucht. Die wäßrige, den Katalysator enthaltende Phase, wird erneut zur Hydroformylierung derselben Menge 1-Hexen unter den vorstehend beschriebenen Bedingungen verwendet.

a) Die GC/MS-Analyse der farblosen organischen Phase ergibt 84 % Umsatz von 1-Hexen zu Heptanal(1) bei einem n/i-Verhältnis von 95 : 5.
b) Die GC/MS-Analyse der organischen Phase ergibt 78 % Umsatz von 1-Hexen zu Heptanal(1) bei einem n/i-Verhältnis von 94 : 6.
c) Die GC/MS-Analyse der organischen Phase ergibt 75 % Umsatz von 1-Hexen zu Heptanal(1) bei einem n/i-Verhältnis von 90 : 10.
d) Die GC/MS-Analyse der organischen Phase ergibt 74 % Umsatz von 1-Hexen zu Heptanal(1) bei einem n/i-Verhältnis von 84 : 16.

Beispiel 45: Hydroformylierung von 1-Hexen in Gegenwart von $IrH(CO)(TPPTS)_3 \cdot 9\,H_2O$ als Katalysator

In einem 50 ml-Laborautoklaven werden unter Stickstoffatmosphäre 63 mg (0.03 mmol) $IrH(CO)(TPPTS)_3 \cdot 9\,H_2O$ und 0.51 g (0.9 mmol) TPPTS in 3 ml Wasser vorgelegt. Nach Zugabe von 2.52 g (30 mmol) 1-Hexen preßt man $CO/H_2$ bis zu einem Druck von 5 MPa auf und erhitzt das Zweiphasensystem auf 110 °C, wobei der Druck auf 6,5 MPa steigt. Nach 20 h Reaktion bei dieser Temperatur läßt man auf Raumtemperatur abkühlen und trennt die Phasen. Die GC/MS-Analyse der organischen Phase ergibt, daß der Umsatz von 1-Hexen zu Heptanal(1) 15 % beträgt, bei einem n/i-Verhältnis von 93 : 7.

Beispiel 46: Hydroformylierung von 1-Hexen in Gegenwart von $(cis\text{-}Cl_2)Pt(TPPTS)_2 \cdot 6\,H_2O$ als Katalysator

In einen 50 ml-Laborautoklaven werden unter $N_2$-Schutz 45 mg (0.03 mmol) $cis\text{-}Cl_2Pt(TPPTS)_2 \cdot 6\,H_2O$ in 2 ml Wasser gegeben. Nach Zugabe von 2.43 g (30 mmol) 1-Hexen preßt man bei Raumtemperatur $CO/H_2$ bis zu einem Druck von 7 MPa auf. Man läßt das Zweiphasensystem 18 h bei 100 °C reagieren, wobei der Druck auf 8 MPa steigt. Nach Beendigung der Reaktion kühlt man auf Raumtemperatur ab und trennt die Phasen. Die farblose organische Phase wird durch GC/MS-Analyse untersucht: 58 % des 1-Hexens wurden zu Heptanal(1) hydroformyliert, das n/i-Verhältnis beträgt 2 : 1. Die wäßrige Phase wird erneut mit 2.43 g (30 mmol) 1-Hexen versetzt. Die Hydroformylierung wird unter denselben Bedingungen durchgeführt; man erhält 62 % Heptanal(1) (n/i-Verhältnis 2.2 : 1).

Beispiel 47: Hydroformylierung von 1-Hexen in Gegenwart von $(cis\text{-}Cl_2)Pt(TPPTS)_2 \cdot 6\,H_2O/SnCl_2$ als Katalysator

Ein 50 ml-Laborautoklav wird mit 45 mg (0.03 mmol) $cis\text{-}Cl_2Pt(TPPTS)_2 \cdot 6\,H_2O$ und 7 mg (0.03 mmol) $SnCl_2 \cdot 2\,H_2O$ in 2 ml Wasser beschickt. Nach Zugabe von 2.43 g (30 mmol) 1-Hexen wird $CO/H_2$ bis zu einem Druck von 7 MPa aufgepreßt. Man erhitzt auf 100 °C, wobei der Druck auf 8 MPa steigt und rührt 18 h bei dieser Temperatur. Nach Abkühlen auf Raumtemperatur trennt man die Phasen. Die GC/MS-Analyse der farblosen organischen Phase ergibt, daß 1-Hexen vollständig zu einem Gemisch aus 70 Gew.-% n-Heptanal(1), 26 Gew.-% i-Heptanal(1) und 4 Gew.-% n-Heptanol(1) umgesetzt wurde.

Beispiel 48: Katalytische Oxidation von Cyclohexen mit Iodosobenzol und $RuCl_2(TPPTS)_2 \cdot 6\,H_2O$ als Katalysator

Zu einer Suspension von 0.33 g (1.5 mmol) Iodosobenzol in 5 ml Methylenchlorid gibt man 82 mg (1 mmol) Cyclohexen und 30 mg (0.02 mmol) $RuCl_2(TPPTS)_2 \cdot 6\,H_2O$ in 2 ml Wasser und läßt 5 h zwischen 10 °C und 15 °C rühren. Die Untersuchung der abgetrennten organischen Phase durch GC/MS- und GC/IR-Analyse ergibt, daß Cyclohexen zu 70 % zu Cyclohexenoxid oxidiert wird.

Beispiel 49: Katalytische Oxidation von 3-Hexin mit Iodosobenzol und $RuCl_2(TPPTS)_2 \cdot 6\,H_2O$ als Katalysator

Zu einer Suspension von 0.66 g (3 mmol) Iodosobenzol in 5 ml Methylenchlorid gibt man 82 mg (1 mmol) 3-Hexin und 15 mg (0.01 mmol) $RuCl_2(TPPTS)_2 \cdot 6\,H_2O$ in 2 ml Wasser. Man läßt 3 h bei Raumtemperatur rühren und trennt dann die Phasen. Die Untersuchung der organischen Phase durch GC/MS- und GC/IR-Analyse ergibt, daß 3-Hexin vollständig zu Hexan-3.4-dion oxidiert $[v(CO) = 1721\ cm^{-1}]$ wird, daneben läßt sich noch Iodbenzol nachweisen.

Beispiel 50: Katalytische Oxidation von Diphenylacetylen mit Iodosobenzol und RuCl$_2$(TPPTS)$_2$ · 6 H$_2$O als Katalysator

Zu einer Suspension von 660 mg (3 mmol) Iodosobenzol in 4 ml Methylenchlorid gibt man eine Lösung von 180 mg (1.0 mmol) Diphenylacetylen in 2 ml Methylenchlorid und eine Lösung von 15 mg (0.01 mmol) RuCl$_2$(TPPTS)$_2$ · 6 H$_2$O in 2 ml Wasser. Man läßt 2 h bei Raumtemperatur rühren, die wäßrige Phase färbt sich grün. Nach Trennung der Phasen wird die mittlerweile klare organische Phase durch GC/MS- und GC/IR-Analyse untersucht; es zeigt sich, daß Diphenylacetylen vollständig zu Diphenylglyoxal (Benzil) [v(CO) = 1692 cm$^{-1}$] oxidiert wird; daneben läßt sich noch Iodbenzol nachweisen. Die wäßrige Phase wird erneut mit 180 mg (1 mmol) Diphenylacetylen und 660 mg (3 mmol) Iodosobenzol in 6 ml Methylenchlorid versetzt. Nach weiteren 2 h Reaktion bei Raumtemperatur werden die Phasen getrennt, die Untersuchung der organischen Phase durch GC/MS-Analyse ergibt, daß Diphenylacetylen vollständig zu Benzil oxidiert wird.

Beispiel 51: Katalytische Oxidation von 1-Phenylethanol(1) mit RuCl$_2$(TPPTS)$_2$ · 6 H$_2$O als Katalysator

Zu einer Suspension von 0.33 g (1.5 mmol) Iodosobenzol in 5 ml Methylenchlorid gibt man 0.12 g (1 mmol) 1-Phenylethanol(1) und 30 mg (0.02 mmol) RuCl$_2$(TPPTS)$_2$ · 6 H$_2$O in 2 ml Wasser. Die wäßrige Phase färbt sich rasch dunkelgrün. Man läßt 3 h bei Raumtemperatur reagieren und isoliert dann die organische Phase. Die Untersuchung der organischen Phase durch GC/MS- und GC/IR-Analyse ergibt, daß 1-Phenylethanol(1) vollständig zu Acetophenon [v(C=O) = 1705 cm$^{-1}$] oxidiert wird.

Beispiel 52: Katalytische Oxidation von Cyclohexanol mit RuCl$_2$(TPPTS)$_2$ · 6 H$_2$O als Katalysator

Zu einer Suspension von 0.33 mg (1.5 mmol) Iodosobenzol in 5 ml Methylenchlorid gibt man 100 mg (1 mmol) Cyclohexanol und 30 mg (0.02 mmol) RuCl$_2$(TPPTS)$_2$ · 6 H$_2$O in 2 ml Wasser. Man läßt 3 h bei Raumtemperatur reagieren und isoliert dann die organische Phase. Die GC/MS-Analyse ergibt, daß Cyclohexanol vollständig zu Cyclohexanon oxidiert wird.

Beispiel 53: Katalytische Oxidation von 1-Phenylethanol(1) mit Iodosobenzol und Pd(TPPTS)$_3$ · 9 H$_2$O als Katalysator

Zu einer Suspension von 1.32 g (6 mmol) Iodosobenzol in 5 ml Methylenchlorid gibt man 49 mg (4 mmol) 1-Phenylethanol(1) und 80 mg (0.04 mmol) Pd(TPPTS)$_3$ · 9 H$_2$O in 3 ml Wasser und läßt 15 h bei Raumtemperatur rühren. Die Untersuchung der organischen Phase durch GC/MS-Analyse ergibt, daß 35 % des 1-Phenylethanol(1) zu Acetophenon oxidiert werden.

Beispiel 54: Katalytische Oxidation von Triphenylphosphan mit Pd(TPPTS)$_3$ · 9 H$_2$O als Katalysator

1) Zu 2.62 g (10 mmol) Triphenylphosphan in 15 ml Toluol gibt man 60 mg (0.03 mmol) Pd(TPPTS)$_3$ · 9 H$_2$O in 4 ml Wasser und rührt das entstehende Zweiphasengemisch 90 min kräftig an der Luft bei Raumtemperatur. Anschließend wird die organische Phase durch Phasentrennung isoliert und über wasserfreiem Natriumsulfat getrocknet. Das Lösungsmittel wird im Vakuum einer Wasserstrahlpumpe abgezogen. Aus dem $^{31}$P-NMR-Spektrum folgt, daß 86 % des Phosphans zu Triphenylphosphanoxid oxidiert wird.

2) Zu einer Lösung von 2.62 g (10 mmol) Triphenylphosphan in 15 ml Toluol gibt man bei Raumtemperatur 60 mg (0.03 mmol) Pd(TPPTS)$_3$ · 9 H$_2$O in 4 ml Wasser und leitet durch das entstehende Zweiphasengemisch 90 min lang Sauerstoff. Die organische Phase wird durch Phasentrennung isoliert und über wasserfreiem Natriumsulfat getrocknet. Nach Abziehen des Lösungsmittels kristallisiert man den Rückstand aus Toluol um. Ausbeute: 2.77 g (99 %) O=P(C$_6$H$_5$)$_3$; farblose Kristalle.

Charakterisierung

$^{31}$P-NMR (109.3 MHz, C$_7$D$_8$, 20 °C): δ = 26.3 ppm (s)
IR (KBr, cm$^{-1}$): v(P=O) = 1187 (sst), 1117 (sst)

Beispiel 55: Katalytische CC-Kupplung in Gegenwart von Pd(TPPTS)$_3$/CuI als Katalysator

a) Unter Rühren gibt man 40 mg (0.20 mmol) Kupfer(I)-iodid zu einer Mischung aus 10 ml Diethylamin, 1.78 g (11 mmol) 3-Ethyl-3-methyl-1-bromallen, 1.02 g (10 mmol) Phenylacetylen und 200 mg (0.1 mmol) Pd(TPPTS)$_3$ · 9 H$_2$O. Nach 15 h Rühren bei Raumtemperatur setzt man 3 ml Wasser zu und zieht das Diethylamin im Ölpumpenvakuum ab. Der Rückstand wird dreimal mit je 30 ml n-Pentan extrahiert und dann dreimal mit je 30 ml einer gesättigten wäßrigen Natriumchlorid-Lösung gewaschen. Nach dem Trocknen über wasserfreiem Natriumsulfat wird das Lösungsmittel im Vakuum abgezogen. Die Reinigung des Produktes 2-Ethyl-6-phenylhexadien(2.3)-in(5), $CH_3C(C_2H_5)C=C=C(H)C\equiv C(C_6H_5)$, erfolgt durch Chromatographie über Kieselgel (0.063 - 0.200 mm) Petrolether-Diethylether (50 : 1) oder durch Destillation im rotierenden Kugelrohr. Ausbeute 1.10 g (60 %, bezogen auf Phenylacetylen), gelbes Öl.

b) Unter Rühren gibt man 30 mg (0.16 mmol) Kupfer(I)-iodid zu einer Mischung, aus 200 mg NaOH (5 mmol) in 10 ml Wasser, 680 mg (4.2 mmol) 3-Ethyl-3-methyl-1-bromallen, 400 mg (4.0 mmol) Phenylacetylen und 160 mg (0.08 mmol) $Pd(TPPTS)_3 \cdot 9\ H_2O$. Man läßt 40 h bei Raumtemperatur rühren und extrahiert anschließend dreimal mit je 25 ml n-Pentan. Die organische Phase wird dreimal mit je 20 ml gesättigter Natriumchlorid-Lösung gewaschen und über wasserfreiem Natriumsulfat getrocknet, darauf zieht man das Lösungsmittel im Vakuum ab. Ausbeute nach Reinigung: 100 mg (14 %, bezogen auf Phenylacetylen).

## Charakterisierung

$^1$H-NMR  (270 MHz, 25 °C, $CDCl_3$): $\delta$ = 1.03 ppm (t, 3 H, $^3J$ = 7.5 Hz) $CH_2CH_3$, $\delta$ = 1.75 ppm (d, 3 H, $^5J$ = 2.9 Hz) = $C(CH_3)$, $\delta$ = 1.97 - 2.14 ppm (m, 2H) $CH_2CH_3$, $\delta$ = 5.52 (sext. 1 H, $^5J$ = 2.8 Hz), $\delta$ = 7.22 - 7.42 ppm (m, 5H) $C_6H_5$.

IR  (Film, $cm^{-1}$): $v(C\equiv C)$ = 2207, $v(C=C=C)$ = 1947

c) Unter Rühren gibt man 30 mg (0.16 mmol) Kupfer(I)iodid zu einer Mischung aus 0.2 g (5 mmol) Natriumhydroxid und 0.16 g (0.08 mmol) $Pd(TPPTS)_3 \cdot 9\ H_2O$ in 10 ml Wasser. Anschließend fügt man 0.68 g (4.2 mmol) 3-Ethyl-3-methyl-1-bromallen und 0.4 g (4 mmol) Phenylacetylen in 10 ml Pentan zu. Man läßt 20 h bei Raumtemperatur rühren und trennt dann die Phasen. Die wäßrige Phase wird zweimal mit je 15 ml Pentan extrahiert. Die organische Phase wird dreimal mit je 20 gesättigter Natriumchlorid-Lösung gewaschen und über wasserfreiem Natriumsulfat getrocknet. Darauf zieht man das Lösungsmittel im Vakuum ab. Ausbeute nach Reinigung: 0.25 g (35 %, bezogen auf Phenylacetylen); gelbes Öl.

## Charakterisierung

$^1$H-NMR  (270 MHz, 25 °C, $CDCl_3$): $\delta$ = 1.03 ppm (t, 3H, $^3J$ = 7.5 Hz) $CH_2CH_3$
$\delta$ = 1.75 ppm (c, 3H, $^5J$ = 2.9 Hz) = $C(CH_3)$
$\delta$ = 1.97-2.14 ppm (m, 2H) $CH_2CH_3$
$\delta$ = 5.52 ppm (sext. 1H, $^5J$ = 2.8 Hz) = $C(H)$
$\delta$ = 7.22-7.42 ppm (m, 5H) $C_6H_5$

IR  (Film. $cm^{-1}$): $(C\equiv C)$ = 2207, $v(C=C=C)$ = 1947

## Beispiel 56: Amin-Addition an eine CC-Doppelbindung

In einem 15 min mit Stickstoff gespülten 50 ml-Laborautoklaven werden eine Lösung von 60 mg (0.04 mmol) $PtCl_2(TPPTS)_2 \cdot 6\ H_2O$ in 3 ml Wasser, 4.08 g (60 mmol) Isopren und 2.93 g (40 mmol) Diethylamin vorgegeben. Anschließend erwärmt man 2 d auf 80 °C, es stellt sich ein Druck von 0.2-0.3 MPa ein. Nach Abkühlen auf Raumtemperatur trennt man die Phasen und trocknet die organische Phase über wasserfreiem Natriumsulfat. Nach GC/MS-Analyse erhält man 1-(N,N-Diethylamino)-3-methylbuten(2), $(CH_2)_2C=CHCH_2N(C_2H_5)_2$ in 69 % Ausbeute [$v(C=CH)$ = 1670 $cm^{-1}$; $\delta$ (=CH= = 843 $cm^{-1}$]. Ferner entstehen 22 % 1-(N,N-Diethylamino)-2-methylbuten(2), $CH_3CH = C(CH_3)$-$CH_2N(C_2H_5)_2$, in 22 % Ausbeute [$v(C=CH)$ = 1653 $cm^{-1}$; $\delta$ (=CH) = 823 $cm^{-1}$].

## Patentansprüche

1. Komplexverbindungen von Elementen der Gruppe VIII A des Periodensystems, ausgenommen die Verbindungen $RhCl(P(m-C_6H_4SO_3Na)_3)_3$, $RhCl(P(m-C_6H_4SO_3Na)_3)_2$, $[RhCl(P(m-C_6H_4SO_3Na)_3)_2]_2$, das Umsetzungsprodukt von Bis(1,5-cyclooctadien)nickel mit dem Trinatriumsalz von Tris(m-sulfophenyl)phosphan und Verbindungen $[Rh(\mu-S-R)(L)(P(m-C_6H_4SO_3Na)_3]_2$, wobei die Reste R, gleich oder verschieden, ein Kohlenwasserstoff-Radikal mit gegebenenfalls einem oder mehreren Substituenten bedeuten oder auch gemeinsam durch ein einziges divalentes Radikal gebildet werden und L CO oder $P(m-C_6H_4SO_3Na)_3$ darstellt, dadurch gekennzeichnet, daß sie als Komplexligand das Trinatriumsalz von Tris(m-sulfophenyl)phosphan und gegebenenfalls weitere Liganden enthalten.

2. Komplexverbindungen nach Anspruch 1, dadurch gekennzeichnet, daß sie der allgemeinen Formel

$$L_w^1 \, L_x^2 \, M_y[P(C_6H_4\text{-m-}SO_3Na)_3]_z$$

folgen, wobei $L^1$ und $L^2$ gleiche oder unterschiedliche Liganden, die neben dem Trinatriumsalz von Tris(m-sulfophenyl)phosphan an das Zentralatom gebunden sein können, bedeuten, M fur die Elemente der Gruppe VIII A des Periodensystems als Zentralatom steht, w, x, y und z ganze Zahlen sind, wobei w und x jeweils 0 bis 7y und y 1 bis 6 bedeuten und z ≤4y ist.

3. Komplexverbindungen nach Anspruch 2, dadurch gekennzeichnet, daß sie neben dem Trinatriumsalz von Tris(m-sulfophenyl)phosphan als weitere Liganden H, CO, NO, $PF_3$, S, Halogen, $\pi$-Aromaten-Liganden, $\pi$-Olefin-Liganden und/oder $\pi$-Acetylen-Liganden enthalten.

4. Komplexverbindungen nach Anspruch 3, dadurch gekennzeichnet, daß der $\pi$-Aromaten-Ligand Cyclopentadienyl ist.

5. Komplexverbindungen nach Anspruch 3, dadurch gekennzeichnet, daß der $\pi$-Olefin-Ligand Cyclooctadien ist.

6. Komplexverbindungen nach Anspruch 3, dadurch gekennzeichnet, daß der $\pi$-Acetylen-Ligand Diphenylacetylen ist.

7. Komplexverbindungen nach Anspruch 1 oder 2, dadurch gekennzeichnet, daß das Zentralatom Eisen, Ruthenium, Kobalt, Rhodium, Iridium, Nickel, Palladium oder Platin ist.

8. Verbindungen nach Anspruch 1 oder 2, dadurch gekennzeichnet, daß sie
Eisenverbindungen $Fe(CO)_4[P(C_6H_4\text{-m-}SO_3Na)_3]$, $Fe(CO)_3[P(C_6H_4\text{-m-}SO_3Na)_3]_2$, $Fe_4(CO)_{11}[P(C_6H_4\text{-m-}SO_3Na)_3]$ oder
Rutheniumverbindungen $Ru(NO)_2[P(C_6H_4\text{-m-}SO_3Na)_3]_2$, $RuCl_2[P(C_6H_4\text{-m-}SO_3Na)_3]_2$ oder
Cobaltverbindungen $Co_2(CO)_6[P(C_6H_4\text{-m-}SO_3Na)_3]_2$, $CoH(CO)[P(C_6H_4\text{-m-}SO_3Na)_3]_3$, $CoH_2[P(C_6H_4\text{-m-}SO_3Na)_3]_3$, $Co_2(CO)_4(H_5C_6\text{-}C{\equiv}C\text{-}C_6H_5)[P(C_6H_4\text{-m-}SO_3Na)_3]_2$ oder
Rhodiumverbindungen $RhCl[P(C_6H_4\text{-m-}SO_3Na)_3]_3$, $Rh(NO)[P(C_6H_4\text{-m-}SO_3Na)_3]_3$, $Rh(CH_3COO)[P(C_6H_4\text{-m-}SO_3Na)_3]_3$, $Rh(CO)(OH)[P(C_6H_4\text{-m-}SO_3Na)_3]_2$, $Rh(CO)Cl[P(C_6H_4\text{-m-}SO_3Na)_3]_2$, $Rh(\mu\text{-}Cl)(CO)[P(C_6H_4\text{-m-}SO_3Na)_3]_2$, $Rh(OH)[P(C_6H_4\text{-m-}SO_3Na)_3]_3$, $Rh_2[P(C_6H_4\text{-m-}SO_3Na)_3]_2[P(C_6H_4\text{-m-}SO_3Na)_2(C_6H_4\text{-m-}SO_3)]_2$, $Rh_2(\mu\text{-}Cl_2)(\eta^4\text{-}C_8H_{12})[P(C_6H_4\text{-m-}SO_3Na)_3]_2$, $Rh_6(CO)_7[P(C_6H_4\text{-m-}SO_3Na)_3]_9$ oder
Iridiumverbindungen $Ir(NO)[P(C_6H_4\text{-m-}SO_3Na)_3]_3$, $IrCl(CO)[P(C_6H_4\text{-m-}SO_3Na)_3]_3$, $IrH(CO)[P(C_6H_4\text{-m-}SO_3Na)_3]_3$, $Ir(\mu\text{-}Cl)(\eta^4\text{-}C_8H_{12})[P(C_6H_4\text{-m-}SO_3Na)_3]_2$ oder
Nickelverbindungen $Ni[P(C_6H_4\text{-m-}SO_3Na)_3]_3$, $Ni(CO)_2[P(C_6H_4\text{-m-}SO_3Na)_3]_2$, $Ni(PF_3)_2[P(C_6H_4\text{-m-}SO_3Na)_3]_2$,
die Palladiumverbindung $Pd[P(C_6H_4\text{-m-}SO_3Na)_3]_3$ oder
Platinverbindungen $Pt[P(C_6H_4\text{-m-}SO_3Na)_3]_4$, $PtCl_2[P(C_6H_4\text{-m-}SO_3Na)_3]_2$ oder
darstellen.

9. Verwendung der Verbindungen nach einem oder mehreren der Ansprüche 1 bis 8 als Hydrierkatalysatoren.

10. Verwendung der Verbindungen nach einem oder mehreren der Ansprüche 1 bis 8 als Katalysatoren für die Einstellung des Wassergasgleichgewichts.

11. Verwendung der Verbindungen nach einem oder mehreren der Ansprüche 1 bis 8 als Hydrocarbonylierungskatalysatoren.

12. Verwendung der Verbindungen nach einem oder mehreren der Ansprüche 1 bis 8 als Hydroformylierungskatalysatoren.

13. Verwendung der Verbindungen nach einem oder mehreren der Ansprüche 1 bis 8 als Oxidationskatalysatoren.

14. Verwendung der Verbindungen nach einem oder mehreren der Ansprüche 1 bis 8 als Katalysatoren für die Verknüpfung von C-C-Doppelbindungen mit C-C-Mehrfachbindungen.

15. Ausführungsform nach Anspruch 14 dadurch gekennzeichnet, daß die Verbindungen als Katalysatoren für die Allen/Alkin-Kupplung verwendet werden.

16. Verwendung der Verbindungen nach einem oder mehreren der Ansprüche 1 bis 8 als Katalysatoren für die Addition von sekundären Aminen an C-C-Doppelbindungen.

## Claims

1. Complex compounds of elements of group VIII A of the periodic table, with the exception of the compounds $RhCl(P(m\text{-}C_6H_4SO_3Na)_3)_3$, $RhCl(P(m\text{-}C_8H_4SO_3Na)_3)_2$, $[RhCl(P(m\text{-}C_6H_4SO_3Na)_3)_2]_2$, the reaction product of bis(1,5-cyclooctadiene)nickel with the trisodium salt of tris(m-sulfophenyl)phosphane and compounds $[Rh(\mu\text{-}S\text{-}R)(L)(P(m\text{-}C_6H_4SO_3Na)_3)]_2$, where the groups R, either the same or different, denote a hydrocarbon radical with optionally one or more substituents or together are formed by one single divalent radical and L represents CO or $P(m\text{-}C_6H_4SO_3Na)_3$, characterised in that they contain the trisodium salt of tris(m-sulfophenyl)phosphane as the complex ligand and optionally other ligands.

2. Complex compounds according to claim 1, characterised in that they correspond to the general formula

$$L^1_w L^2_x M_y [P(C_6H_4\text{-}m\text{-}SO_3Na)_3]_z$$

where $L^1$ and $L^2$ denote the same or different ligands which, in addition to the trisodium salt of tris(m-sulfophenyl)phosphane, can be bound to the central atom, M stands for the elements of group VIII A of the periodic table as the central atom, w, x, y and z are integers, w and x each being 0 to 7y, y being 1 to 6 and z being $\leq 4y$.

3. Complex compounds according to claim 2, characterised in that, in addition to the trisodium salt of tris(m-sulfophenyl)phosphane, they contain H, CO, NO, $PF_3$, S, halogen, $\pi$-aromatic ligands, $\pi$-olefin ligands and/or $\pi$-acetylene ligands as other ligands.

4. Complex compounds according to claim 3, characterised in that the $\pi$-aromatic ligand is cyclopentadienyl.

5. Complex compounds according to claim 3, characterised in that the $\pi$-olefin ligand is cyclooctadiene.

6. Complex compounds according to claim 3, characterised in that the $\pi$-acetylene ligand is diphenylacetylene.

7. Complex compounds according to claim 1 or 2, characterised in that the central atom is iron, ruthenium, cobalt, rhodium, iridium, nickel, palladium or platinum.

8. Compounds according to claim 1 or 2, characterised in that they represent
iron compounds $Fe(CO)_4[P(C_6H_4\text{-}m\text{-}SO_3Na)_3]$, $Fe(CO)_3[P(C_6H_4\text{-}m\text{-}SO_3Na)_3]_2$, $Fe_4(CO)_{11}[P(C_6H_4\text{-}m\text{-}SO_3Na)_3]$ or
ruthenium compounds $Ru(NO)_2[P(C_6H_4\text{-}m\text{-}SO_3Na)_3]_2$, $RuCl_2[P(C_6H_4\text{-}m\text{-}SO_3Na)_3]_2$ or
cobalt compounds $Co_2(CO)_6[P(C_6H_4\text{-}m\text{-}SO_3Na)_3]_2$, $CoH(CO)[P(C_6H_4\text{-}m\text{-}SO_3Na)_3]_3$, $CoH_2(P(C_6H_4\text{-}m\text{-}SO_3Na)_3)_3$, $Co_2(CO)_4(H_5C_8\text{-}C\equiv C\text{-}C_6H_5)[P(C_6H_4\text{-}m\text{-}SO_3Na)_3]_2$ or
rhodium compounds $RhCl[P(C_6H_4\text{-}m\text{-}SO_3Na)_3]_3$, $Rh(NO)(P(C_6H_4\text{-}m\text{-}SO_3Na)_3)_3$, $Rh(H_3COO)[P(C_6H_4\text{-}m\text{-}SO_3Na)_3]_3$, $Rh(CO)(OH)[P(C_6H_4\text{-}m\text{-}SO_3Na)_3]_2$, $Rh(CO)Cl[P(C_6H_4\text{-}m\text{-}SO_3Na)_3]_2$, $Rh(\mu\text{-}Cl)(CO)[P(C_6H_4\text{-}m\text{-}SO_3Na)_3]_2$, $Rh(OH)[P(C_6H_4\text{-}m\text{-}SO_3Na)_3]_3$, $Rh_2[P(C_6H_4\text{-}m\text{-}SO_3Na)_3]_2[P(C_6H_4\text{-}m\text{-}SO_3Na)_2(C_6H_4\text{-}m\text{-}SO_3)]_2$, $Rh_2(\mu\text{-}Cl_2)(\eta^4\text{-}C_8H_{12})[P(C_6H_4\text{-}m\text{-}SO_3Na)_3]_2$, $Rh_6(CO)_7[P(C_6H_4\text{-}m\text{-}SO_3Na)_3]_9$, or
iridium compounds $Ir(NO)[P(C_6H_4\text{-}m\text{-}SO_3Na)_3]_3$, $IrCl(CO)[P(C_6H_4\text{-}m\text{-}SO_3Na)_3]_3$, $IrH(CO)[P(C_6H_4\text{-}m\text{-}SO_3Na)_3]_3$, $Ir(\mu\text{-}Cl)(\eta^4\text{-}C_8H_{12})[P(C_6H_4\text{-}m\text{-}SO_3Na)_3]_2$, or
nickel compounds $Ni[P(C_6H_4\text{-}m\text{-}SO_3Na)_3]_3$, $Ni(CO)_2[P(C_6H_4\text{-}m\text{-}SO_3Na)_3]_2$, $Ni(PF_3)_2[P(C_6H_4\text{-}m\text{-}SO_3Na)_3]_2$,
the palladium compound $Pd[P(C_6H_4\text{-}m\text{-}SO_3Na)_3]_3$ or
platinum compounds $Pt[P(C_6H_4\text{-}m\text{-}SO_3Na)_3]_4$, $PtCl_2[P(C_6H_4\text{-}m\text{-}SO_3Na)_3]_2$.

9. Use of the compounds according to one or more of claims 1 to 8 as hydrogenation catalysts.

10. Use of the compounds according to one or more of claims 1 to 8 as catalysts for adjusting the water gas equilibrium.

11. Use of the compounds according to one or more of claims 1 to 8 as hydrocarbonylation catalysts.

12. Use of the compounds according to one or more of claims 1 to 8 as hydroformylation catalysts.

13. Use of the compounds according to one or more of claims 1 to 8 as oxidation catalysts.

**14.** Use of the compounds according to one or more of claims 1 to 8 as catalysts for cross-linking carbon-carbon double bonds with carbon-carbon multiple bonds.

**15.** Embodiment according to claim 14, characterised in that the compounds are used as catalysts for allene/alkyne coupling.

**16.** Use of the compounds according to one or more of claims 1 to 8 as catalysts for the addition of secondary amines to carbon-carbon double bonds.

**Revendications**

**1.** Complexes d'éléments du groupe VIII A de la classification périodique, à l'exception des composes $RhCl(P(m-C_6H_4SO_3Na)_3)_3$, $RhCl(P(m-C_6H_4SO_3Na)_3)_2$, $[RhCl(P(m-C_6H_4SO_3Na)_3)_2]_2$, du produit de réaction du bis(1,5-cyclooctadiène)nickel avec le sel trisodique du tris(m-sulfophényl)phosphane et des composés $[Rh(\mu\text{-}S\text{-}R)(L)(P(m-C_6H_4SO_3Na)_3]_2$ pour lesquels les symboles R, ayant des significations identiques ou différentes, représentent chacun un radical hydrocarboné portant le cas échéant un ou plusieurs substituants, ou bien les symboles R représentent ensemble un radical divalent unique, et L représente CO ou $P(m-C_6H_4SO_3Na)_3$, caractérisés en ce qu'ils contiennent en tant que ligand des complexes le sel trisodique du tris(m-sulfophényl)phosphane avec, le cas échéant, d'autres ligands.

**2.** Complexes selon revendication 1, caractérisés en ce qu'ils répondent à la formule générale

$$L_w^1 L_x^2 M_y[P(C_6H_4\text{-}m\text{-}SO_3Na)_3]_z$$

dans laquelle $L^1$ et $L^2$ représentent des ligands identiques ou différents qui peuvent être fixés, avec le sel trisodique du tris(m-sulfophényl)phosphane, sur l'atome central, M représente les éléments du groupe VIII A de la classification périodique, qui consituent les atomes centraux, w, x, y et z sont des nombres entiers, w et x ont chacun une valeur de 0 à 7y, y a une valeur de 1 à 6 et $z \leq 4y$.

**3.** Complexes selon revendication 2, caractérisés en ce qu'ils contiennent en tant qu'autres ligands, en plus du sel trisodique du tris(m-sulfophényl)phosphane, H, CO, NO, $PF_3$, S, un halogène, des ligands $\pi$-aromatiques, des ligands $\pi$-oléfiniques et/ou des ligands $\pi$-acétyléniques.

**4.** Complexes selon revendication 3, caractérisés en ce que le ligand $\pi$-aromatique est le ligand cyclopentadiényle.

**5.** Complexes selon revendication 3, caractérisés en ce que le ligand $\pi$-oléfinique est le ligand cyclooctadiène.

**6.** Complexes selon revendication 3, caractérisés en ce que le ligand $\pi$-acétylénique est le diphénylacétylène.

**7.** Complexes selon revendication 1 ou 2, caractérisés en ce que l'atome central est un atome de fer, de ruthénium, de cobalt, de rhodium, d'iridium, de nickel, de palladium ou de platine.

**8.** Composés selon revendication 1 ou 2, caractérisés en ce qu'ils consistent en
les composés du fer $Fe(CO)_4[P(C_6H_4\text{-}m\text{-}SO_3Na)_3]$, $Fe(CO)_3[P(C_6H_4\text{-}m\text{-}SO_3Na)_3]_2$, $Fe_4(CO)_{11}$ $[P(C_6H_4\text{-}m\text{-}SO_3Na)_3]$ ou bien
les composés du ruthénium $Ru(NO)_2[P(C_6H_4\text{-}m\text{-}SO_3Na)_3]_2$, $RuCl_2[P(C_6H_4\text{-}m\text{-}SO_3Na)_3]_2$ ou bien
les composés du cobalt $Co_2(CO)_6[P(C_6H_4\text{-}P(SO_3Na)_3]_2$, $CoH(CO)[P(C_6H_4\text{-}m\text{-}SO_3Na)_3]_3$, $CoH_2[P(C_6H_4\text{-}m\text{-}SO_3Na)_3]_3$, $Co_2(CO)_4(H_5C_6\text{-}C{\equiv}C\text{-}C_6H_5)[P(C_6H_4\text{-}m\text{-}SO_3Na)_3]_2$ ou bien
les composés du rhodium $RhCl[P(C_6H_4\text{-}m\text{-}SO_3Na)_3]_3$, $Rh(No)[P(C_6H_4\text{-}m\text{-}SO_3Na)_3]_3$, $Rh(CH_3COO)[P(C_6H_4\text{-}m\text{-}SO_3Na)_3]_3$, $Rh(CO)(OH[P(C_6H_4\text{-}m\text{-}SO_3Na)_3]_2$, $Rh(CO)Cl[P(C_6H_4\text{-}m\text{-}SO_3Na)_3]_2$, $Rh(\mu\text{-}Cl)(CO)[P(C_6H_4\text{-}m\text{-}SO_3Na)_3]_2$, $Rh(OH)[P(C_6H_4\text{-}m\text{-}SO_3Na)_3]_3$, $Rh_2[P(C_6H_4\text{-}m\text{-}SO_3Na)_3]_2[P(C_6H_4\text{-}m\text{-}SO_3Na)_2(C_6H_4\text{-}m\text{-}SO_3)]_2$, $Rh_2(\mu\text{-}Cl_2)(\eta^4\text{-}C_8H_{12})[P(C_6H_4\text{-}m\text{-}SO_3Na)_3]_2$, $Rh_6(CO)_7[P(C_6H_4\text{-}m\text{-}SO_3Na)_3]_9$ ou bien
les composés de l'iridium $Ir(NO)[P(C_6H_4\text{-}m\text{-}SO_3Na)_3]_3$, $IrCl(CO)[P(C_6H_4\text{-}m\text{-}SO_3Na)_3]_3$, $IrH(CO)[P(C_6H_4\text{-}m\text{-}SO_3Na)_3]_3$, $Ir(\mu\text{-}Cl)(\eta^4\text{-}C_8H_{12})[P(C_6H_4\text{-}m\text{-}SO_3Na)_3]_2$ ou bien
les composés du nickel $Ni[P(C_6H_4\text{-}m\text{-}SO_3Na)_3]_3$, $Ni(CO)_2[P(C_6H_4\text{-}m\text{-}SO_3Na)_3]_2$, $Ni[PF_3]_2[P(C_6H_4\text{-}m\text{-}SO_3Na)_3]_2$, le composé de palladium $Pd[P(C_6H_4\text{-}m\text{-}SO_3Na)_3]_3$ ou bien
les composés du platine $Pt[P(C_6H_4\text{-}m\text{-}SO_3Na)_3]_4$, $PtCl_2[P(C_6H_4\text{-}m\text{-}SO_3Na)_3]_2$.

**9.** Utilisation des composés selon une ou plusieurs des revendications 1 à 8 en tant que catalyseurs d'hydrogénation.

**10.** Utilisation des composés selon une ou plusieurs des revendications 1 à 8, en tant que catalyseurs pour le réglage de l'équilibre du gaz à l'eau.

**11.** Utilisation des composés selon une ou plusieurs des revendications 1 à 8 en tant que catalyseurs d'hydrocarbonylation.

**12.** Utilisation des composés selon une ou plusieurs des revendications 1 à 8 en tant que catalyseurs d'hydroformylation.

**13.** Utilisation des compsoés selon une ou plusieurs des revendications 1 à 8 en tant que catalyseurs d'oxydation.

**14.** Utilisation des compsoés selon une ou plusieurs des revendications 1 à 8 en tant que catalyseurs pour la condensation de doubles liaisons C-C avec des liaisons multiples C-C.

**15.** Mode de réalisation selon la revendication 14, caractérisé en ce que les composés sont utilisés en tant que catalyseurs pour la condensation allène/alcyne.

**16.** Utilisation des compsoés selon une ou plusieurs des revendications 1 à 8 en tant que catalyseurs pour la fixation par addition d'amines secondaires sur des doubles liaisons C-C.